# EUROPÄISCHE PATENTSCHRIFT

(11) **EP 0 516 655 B1**
(45) Veröffentlichungstag und Bekanntmachung des Hinweises auf die Patenterteilung: **04.05.1994**
(21) Anmeldenummer: 91903789.5
(22) Anmeldetag: 19.02.1991
(51) Int. Cl.: C12N 15/62, A61K 39/00

(54) **TRÄGERGEBUNDENE REKOMBINANTE PROTEINE, VERFAHREN ZUR HERSTELLUNG UND VERWENDUNG ALS IMMUNOGENE UND VAKZINE**
CARRIER-BOUND RECOMBINANT PROTEIN, PROCESS FOR PRODUCING IT AND ITS USE AS AN IMMUNOGEN AND VACCINE
PROTEINE RECOMBINANTE IMMOBILISEE SUR UN SUBSTRAT, SON PROCEDE DE PRODUCTION ET SON UTILISATION COMME AGENT IMMUNOGENE ET COMME VACCIN

(30) Priorität: 24.02.1990 DE 4005874
(43) Veröffentlichungstag der Anmeldung: 09.12.1992
(73) Patentinhaber: Lubitz, Werner, Dr., 1030 Wien (AT)
(72) Erfinder: LUBITZ, Werner, D-8000 München 2 (DE); SZOSTAK, Michael, P., D-8000 München 40 (DE)
(74) Vertreter: Huber, Bernhard, Dipl.-Chem.
(86) Internationale Anmeldenummer: EP9100308
(87) Internationale Veröffentlichungsnummer: WO9113155

(56) Entgegenhaltungen:
- EP-A- 0 291 021
- WO-A-87/06590
- Res. Microbiol., Band 141, Nr. 7-8, 1990, Institut Pasteur/ Elsevier, (Paris, FR), M. Szostak et al., S. 1005-1007

## Beschreibung

Die Erfindung betrifft trägergebundene rekombinante Proteine, ein Verfahren zu ihrer Herstellung und ihre Verwendung, insbesondere als Immunogene und Vakzine.

Die Hauptaufgabe des Immunsystems bei Mensch und Tier besteht in der Abwehr und Vermeidung pathologischer Schäden, die aufgrund von entarteten Zellen infektiösen Viren, Bakterien, Pilzen oder Protozoen entstehen. Das Immunsystem zeichnet sich dadurch aus, daß eine immer stärker werdende Resistenz nach wiederholten Infekten mit Krankheitserregern auftritt. Ziel der Immunisierung ist es, Abwehrkräfte des Immunsystems gegen bestimmte Krankheitserreger aufzubauen, ohne entsprechende Krankheiten auszulösen.

Antikörper und zelluläre T- und B-Lymphozyten, sorgen für die spezifische Abwehr von Erregern. Dabei ist die Erkennung fremder Strukturen wie z. B. solcher, die auf einer Bakterienzelle vorkommen, eine wesentliche Voraussetzung. Je nach Stimulierung des Immunsystems kann dabei nach Immunisierung eine zeitlich begrenzte oder eine lebenslange Immunität gegen Krankheitserreger aufgebaut werden.

Für die Qualität von monoklonalen und polyklonalen Antikörpern sowie für die Wirksamkeit von Vakzinen ist es wesentlich, daß die Immunantwort auf das Antigen in ausreichendem Umfang erfolgt. Häufig zeigen jedoch virale Antigene oder rekombinante humane Proteine, wenn sie ohne weitere Modifikation eingesetzt werden, eine schlechte oder gar keine Immunantwort. Aus diesem Grund werden diese Antigene häufig an Träger (vorzugsweise an Proteine) gekoppelt um die Immunantwort zu verstärken. Durch die Bindung der Antigene an den Träger können jedoch die Antigene im oder in der Nähe der antigenen Determinante verändert werden. Dadurch kann die Immunantwort beträchtlich geschwächt werden.

Zur Verbesserung der Immunantwort ist es vorteilhaft, solche Antigene in die äußere Membran von Bakterien einzubauen und diese Komplexe als Immunogene zu verwenden (J. Immunol. 139 (1987) 1658 - 1664, Bacterial Vaccines and Local Immunity - Ann. Sclavor 1986, n.1-2, pp. 19-22, Proceedings of Sclavo International Conference, Siena, Italy, 17-19 November 1986). Auch werden abgeschwächte bzw. abgetötete Erreger (Bakterien oder Viren), aufbereitete Teilkomponenten von Krankheitserregern (Membranproteine von Bakterien, Strukturproteine von Viren) oder rekombinante Lebendvakzine (Viren oder Bakterien) eingesetzt.

Ein Nachteil bei der Verwendung von lebenden Bakterien oder Viren als Immunogene für die Immunisierung liegt darin, daß eine unerwünschte krankheitserregende Ausbreitung der Keime nicht ausgeschlossen werden kann.

Durch Abtötung oder Fragmentierung der Bakterien und Viren vor Verwendung als Immunogen oder Vakzine kann allerdings die antigene Determinante verändert werden, wodurch die Immunantwort deutlich geringer sein kann.

Aufgabe der vorliegenden Erfindung ist es deshalb, Immunogene und Vakzine bereitzustellen, die diese Nachteile nicht besitzen.

Diese Aufgabe wird durch ein trägergebundenes, rekombinantes Protein gelöst, welches erhältlich ist durch Expression einer rekombinanten DNA, in diesen gramnegativen Bakterien, welche eine erste DNA-Sequenz (DNA-Targetsequenz), welche für mindestens eine hydrophobe, nicht lytisch wirkende membranintegrierende Proteindomäne, die eine alphahelicale Struktur besitzt und aus 14 bis 20 Aminosäuren besteht, die N- und C-terminal von je 2 bis 30 beliebigen Aminosäuren flankiert sein können, codiert, eine zweite DNA-Sequenz (DNA-Proteinsequenz), die für ein rekombinantes Protein codiert, sowie eine dritte unter davon getrennter Kontrolle stehenden DNA-Sequenz (DNA-Lysegen), die für ein lytisch wirkendes Membranprotein aus Bacteriophagen oder ein lytisch wirkendes Toxin-release-Gen oder für deren lytisch wirkenden Teile codiert, enthält und Gewinnung des trägergebundenen rekombinanten Proteins aus der Kulturbrühe.

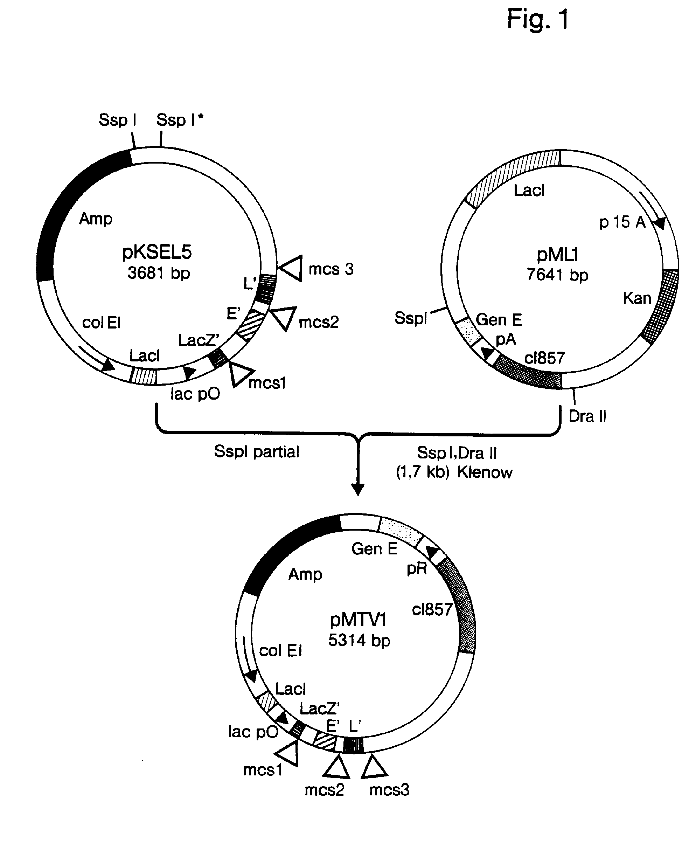

Vorzugsweise wird die Expression des Fusionsprotein-Gens und des Lyse-Gens von zwei verschiedenen Promotoren (Fig.1) aus gesteuert. Die Expression des Lyse-Gens erfolgt vorzugsweise verzögert gegenüber der Expression des Fusionsproteins.

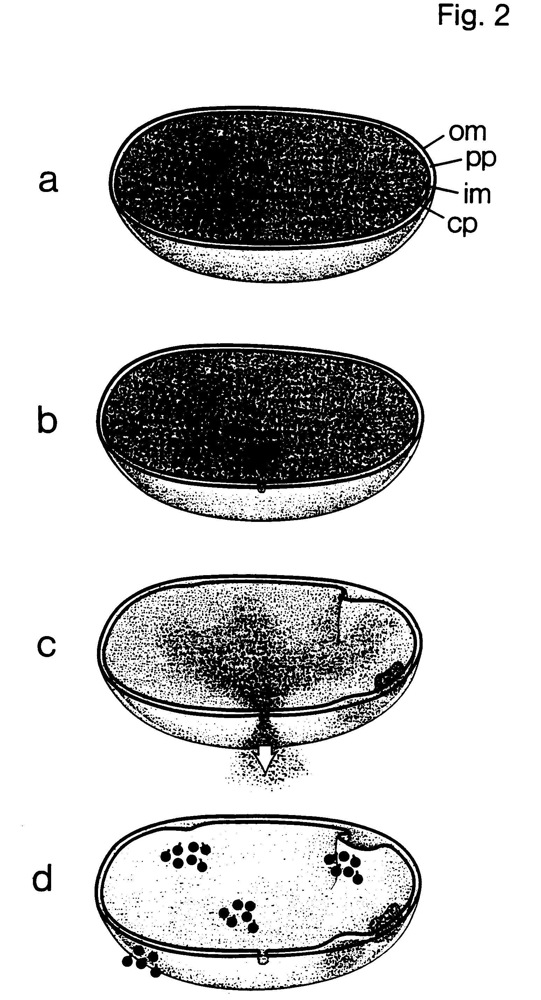

Durch diese Art der Expression von Fusionsprotein-Gen und Lyse-Gen wird erreicht, daß zunächst eine Vielzahl von Fusionsproteinen in die Membran der als Wirtsorganismus verwendeten gramnegativen Bakterien integriert werden und anschließend eine Lyse dieser Bakterien erfolgt. Der sonst dichte Zellwandkomplex der Bakterien wird dabei so permeabilisiert, daß die cytoplasmatischen Bestandteile der Bakterien freigesetzt werden. (Eur. J. Biochem. 180 (1989), 393 - 398). Die Morphologie der Zellen, beispielsweise die Stäbchenform von E.coli Zellen, bleibt erhalten. Es bildet sich lediglich in einem abgegrenzten Bereich der Membran eine Tunnelstruktur aus. Die Tunnelbildung wird begleitet durch eine Fusion der inneren und äußeren Membran am Tunnelrand. Die auf diese Weise entstandenen Bakterienhüllen stellen die Träger für das rekombinante Protein dar und werden im weiteren als Bakterienghosts bezeichnet (Fig. 2).

Die Bakterienghosts bestehen aus cytoplasmatischer (innerer) Membran, periplasmatischem Raum und äußerer Membran, wobei die Integrität des Zellwandkomplexes weitgehend erhalten bleibt. Für Bakterienstämme, die zusätzlich eine S-Layer-Schicht (parakristalline Proteinschicht außerhalb der äußeren Membran) aufweisen, ist diese Proteinschicht ebenfalls Bestandteil der Bakterienghosts (Ann. Rev. Microbiol. 37(1983), 311-339). Die Bakterienghosts sind somit Träger der rekombinanten Proteine (Immunogene) und stellen gleichzeitig aufgrund ihres Aufbaus (Peptidoglykan, Lipopolysaccharid) das Adjuvans zur Verstärkung der Immunantwort dar.

Als Wirtsorganismen sind alle gramnegativen Bakterien, vorzugsweise gramnegative Krankheitserreger wie z.B. Escherichia coli, Bordetella pertussis, Campylobacter nijuni, Corynebacterium diphteriae, Legionella pneumophilia, Listeria monocytogenes, Pseudomonas aeruginosa, Shigella dysenteriae, Vibrio cholerae, Yersinia enterolitica geeignet ( Schaechter, M, H. Medoff, D. Schlesinger, Mechanisms of Microbial Disease. Williams and Wilkins, Baltimore (1989) ).

Die erfindungsgemäßen trägergebundenen, rekombinanten Proteine eignen sich überraschend gut als Immunogene, wobei es zu ausgeprägten Immunantworten und sehr hohen Antikörper-Titern kommt.

Ein besonderer Vorteil ergibt sich dadurch, daß das rekombinante Protein direkt nach der Expression in die Membran der Bakterien integriert und so die Trägerbindung hergestellt wird. Damit erübrigt sich die Isolierung des rekombinanten Proteins als solches vor Herstellung des Immunogens. Da es zudem für die Herstellung von immunogenhaltigen Bakterienghosts ausreichend ist, wenn einige hundert bis zur maximal möglichen Anzahl (ca. 50000) rekombinante Antigene in die Membran der Bakterienghosts integriert sind, erübrigt sich eine Überexpression des rekombinanten Proteins.

Ein weiterer Vorteil des erfindungsgemäßen Verfahrens besteht darin, daß sehr viele antigene Epitope im Zellwandkomplex der Bakterienghosts präsentiert werden. Es hat sich gezeigt, daß die Targetsequenzen fürdie rekombinanten Proteine bestimmte Bereiche innerhalb des Bakterienzellwandkomplexes zur Integration bevorzugen. Diese Bereiche stellen hauptsächlich Adhäsionsstellen der inneren und äußeren Membran dar und stehen mit der Zellteilung der Bakterien in Verbindung. Dadurch kommt es zu keiner uniformen Verteilung des rekombinanten Proteins sondern zu inselartigen Anreicherungen innerhalb des Zellwandkomplexes (vgl. Fig. 2d). Die gehäufte Anordnung der rekombinanten Proteine innerhalb eines relativ kleinen Bereichs (cluster) hat den Vorteil, daß Immunglobulin-tragende B-Zellen zur Poliferation angeregt werden. Zum anderen wirkt das in den Bakterienhüllen vorhandene Lipopolysaccharid als Mitogen und löst ebenfalls ein Signal zur Zellteilung aus. Damit erhält man eine effektive Stimulation der B-Zell-spezifischen Produktion von Immunglobulinen.

Weiter hat sich gezeigt, daß die die erfindungsgemäßen trägergebundenen, rekombinanten Proteine in ihren natürlichen Proteinstrukturen und damit in aktiver Form in die Bakterienmembran integriert sind.

Dies ist besonders überraschend, da üblicherweise rekombinante Proteine nach Expression in Prokaryonten als inclusion bodies (vgl. EP-A0219 874, WO 89/03711) in inaktiver Form erhalten werden und erst anschließend durch Denaturierung und Renaturierung in die aktive Form übergeführt werden können.

Als rekombinante Proteine geeignet sind alle dem Fachmann geläufigen Proteine. Besonders bevorzugt werden Humanproteine und Antigene, insbesondere virale Antigene verwendet. Ihre Größe ist nicht begrenzt. Vorzugsweise beträgt das Molekulargewicht der Antigene aber 2000 bis 200000 Dalton.

Besonders bevorzugt weist das rekombinante Antigen antigene Strukturen von humanen Viren und Retroviren wie z.B. von HIV, HBV und EBV auf.

Die hydrophoben, nicht lytisch wirkenden und membranintegrierenden Proteindomänen werden im weiteren als Targetsequenzen bezeichnet. Bevorzugt sind als Targetsequenzen komplette Sequenzen oder Teilsequenzen von Membranproteinen, die jedoch auch durch Aminosäureaustausche modifiziert sein können. Ein solcher Austausch darf jedoch die Struktur des entsprechenden Proteins nicht verändern.

Vorzugsweise werden solche Targetsequenzen verwendet, die - im Unterschied zu Signalsequenzen von anderen Membranproteinen- durch in der Membran vorkommende Proteasen (z. B. Signalpeptidase und Proteasen des periplasmatischen Raums) nicht gespalten werden. Targetsequenzen können beispielsweise durch Proteinengineering aus natürlich vorkommenden Sequenzen des Lysisgens der PhiX174 Phagengruppe (für N-terminales Targeting) sowie aus den natürlich vorkommenden Sequenzen des Lysisgens der MS2 Phagengruppe (für C-terminales Targeting) abgeleitet werden.

Als Targetsequenz eignet sich vorzugsweise eine hydrophobe alpha-helicale Proteindomäne aus 14 bis 20 Aminosäuren, die N- und C- terminal von jeweils 2 bis 30 beliebigen Aminosäuren flankiert sein kann. Vorzugsweise kann an diese Proteindomäne mindestens eine weitere Proteindomäne gebunden sein. Die Bindung erfolgt vorzugsweise über flexible Linkersequenzen. Unter flexiblen Linkersequenzen sind hydrophile Aminosäuresequenzen mit 2 bis 100 Aminosäuren, vorzugsweise mit 2 bis 30 Aminosäuren, und mit ungeordneter Sekundärstruktur zu verstehen (Turn- und Random Coil-Sequenzen).

Die weiteren Proteindomänen, die an die erste Proteindomäne gekoppelt sind, können analog wie die erste Proteindomäne aufgebaut sein. Es ist jedoch bevorzugt, daß mindestens eine der weiteren Domänen eine ß-Faltblattstruktur besitzt und aus 10 bis 16 Aminosäuren, vorzugsweise 11 bis 13 Aminosäuren, aufgebaut ist. Solche β-Faltblattstrukturen gleichen vorzugsweise in ihrem Aufbau und ihrer Sekundärstruktur ampipathischen Proteinsequenzen, die in Porinen der äußeren Membranen vorkommen. Für ein N-terminales Targeting ist es bevorzugt, solche Targetsequenzen zu verwenden, welche die Aminosäuren 1 bis 54 von Protein E aus dem Phagen PhiX174 enthalten (im weiteren als E'-Sequenz bezeichnet) und nicht lytisch wirken. Für ein C-terminales Targeting ist es bevorzugt, Targetsequenzen zu verwenden, welche die Aminosäuren 21 bis 75 von Protein L aus dem Phagen MS2 enthalten (im weiteren als L'-Sequenz bezeichnet) und nicht lytisch wirken. (Sequenzen vergleiche EP-A 0 291 021). Ebenso geeignet sind Sequenzen, die sich aus den genannten Sequenzen der E- und L-Targetsequenzen durch einen homologen Aminosäureaustausch, der keine Veränderung der Proteinsekundärstruktur verursacht, ableiten.

Unter Membranproteinen von Bacteriophagen sind vorzugsweise Membranproteine von Bacteriophagen der Klasse Mikroviridae, vorzugsweise von icosahedralen, lytischen und ssDNA enthaltenden Phagen, die Enterobacteriacae infizieren können, zu verstehen. Beispiele hierfür sind die Phagen PhiX174, S13, G4, G6, G14, PhiA, PhiB, PhiC, PhiR, welche E. coli C Stämme infizieren können. Ebenso geeignet ist alpha3, welcher E. coli C und E. coli B Stämme infizieren kann. Ebenso geeignet sind die Phagen K9, St-1, PhiK, PhiXtB und U3, welche E. coli K12 Stämme infizieren können (Sinsheimer R.L. (1968) in: Prog. Nucl. Acid Res. Mol. Biol (Davidson J.N. & Cohn W.W., eds) Vol.8, Academic Press, New York & London, pp. 115-169: Tessman E.S. & Tessmann I. (1978) in: The single-stranded DNA Phages (Denhardt D.T., Dressler D. & Ray D.S., eds.) Cold Spring Harbor Press, Cold Spring Harbor, pp. 9-29; Hayashi M., Aoyama A., Richardson D.L. & Hayashi M.N. (1987) in: The Bacteriophages, pp. 1-71).

Als lytisch wirksame Membranproteine sind vorzugsweise Lyseproteine aus den genannten Bakteriophagen sowie andere Toxin-release Gene wie Colicin Lysegen (Microbiol. Sciences 1 (1984) 168-175 und 203 - 205) geeignet.

In einer weiteren bevorzugten Ausführungsform ist an das rekombinante Protein ein nicht kovalent bindender Bindungspartner für dieses Protein, an den gegebenenfalls noch weitere Substanzen kovalent oder nicht kovalent gebunden sind, gebunden. Beispiele für Bindungspaare, deren Partner als Bindungspartner geeignet sind, sind beispielsweise Biotin - Streptavidin bzw. Avidin, Hapten - Antikörper, Antigen - Antikörper, Konkavalin - Antikörper, Zucker - Lectin, Hapten - Bindeprotein (z.B. Thyroxin bindendes Globulin und Thyroxin) oder Oligopeptid-Antikörper.

Bevorzugt wird als bindendes Paar Streptavidin bzw. Avidin und Biotin eingesetzt. Besonders bevorzugt wird als immobilisiertes, rekombinantes Protein Streptavidin bzw. Avidin verwendet und daran biotinyliertes Antigen gebunden.

Weiter ist es bevorzugt als rekombinantes Protein ein Protein zu verwenden, das einen chemischen Liganden erkennt. Beispiele hierfür sind β-Galactosidase/p-Aminophenyl-β-D-thiogalactosid (ein Strukturanaloges der Lactose), Gene 29 (1984) 27-31. Durch die Erkennung des aktiven Zentrums der β-Galactosidase ohne eine Spaltung des Substrats, werden derartig substituierte Produkte an die Bakterienghost gebunden.

Ein weiterer Gegenstand der Erfindung ist eine rekombinante DNA, die eine erste DNA-Sequenz (DNA-Targetsequenz), welche für mindestens eine hydrophobe, nicht lytisch wirkende membranintegrierende Proteindomäne, die eine alphahelicale Struktur besitzt und aus 14 bis 20 Aminosäuren besteht, die N- und C-terminal von je 2 bis 30 beliebigen Aminosäuren flankiert sein können, codiert, eine zweite DNA-Sequenz (DNA-Proteinsequenz), die für ein rekombinantes Protein codiert, sowie eine dritte unter davon getrennter Kontrolle stehenden DNA-Sequenz (DNA-Lysegen), die für ein lytisch wirkendes Membranprotein aus Bacteriophagen oder ein lytisch wirkendes Toxin-release-Gen oder für deren lytisch wirkenden Teile codiert, enthält.

Als DNA-Targetsequenzen werden DNA-Sequenzen bevorzugt, welche für das L'- Protein oder E'- Protein kodieren. Ebenso geeignet sind DNA-Sequenzen, die für von diesen Proteinen abgeleiteten Aminosäuresequenzen mit gleicher Sekundärstruktur kodieren. Diese Sequenzen sind vorzugsweise durch DNA-Sequenzen verbunden, die für hydrophile Proteindomänen mit 2 bis 30 Aminosäuren und ungeordneter Sekundärstruktur codieren.

In einer bevorzugten Ausführungsform enthält die DNA-Lysesequenz die DNA-Sequenz des E-Proteins, die DNA-Sequenz des L-Proteins oder die DNA-Sequenz des EL-Hybridproteins (Sequenzen vgl. EP-A 0 291 021). Ebenso geeignet sind Teilsequenzen davon, die lytisch wirken.

Die DNA-Proteinsequenz ist vorzugsweise die DNA-Sequenz eines viralen Antigens (z. B. HIV, HBV, EBV) oder eines rekombinanten Humanproteins.

Ein weiterer Gegenstand der Erfindung ist ein Verfahren zur Herstellung eines an die Membran von gramnegativen Bakterien gebundenen rekombinanten Proteins, das dadurch gekennzeichnet ist, daß in diesen gramnegativen Bakterien eine rekombinante DNA, die eine erste DNA-Sequenz (DNA-Targetsequenz), welche für mindestens eine hydrophobe, nicht lytisch wirkende membranintegrierende Proteindomäne, die eine alphahelicale Struktur besitzt und aus 14 bis 20 Aminosäuren besteht, die N- und C-terminal von je 2 bis 30 beliebigen Aminosäuren flankiert sein können, codiert, eine zweite DNA-Sequenz (DNA-Proteinsequenz), die für ein rekombinantes Protein codiert, sowie eine dritte unter davon getrennter Kontrolle stehenden DNA-Sequenz (DNA- Lysegen), die für ein lytisch wirkendes Membranprotein aus Bacteriophagen oder ein lytisch wirkendes Toxin-release-Gen oder für deren lytisch wirkenden Teile codiert, enthält exprimiert werden und das trägergebundene rekombinante Protein aus der Kulturbrühe gewonnen wird.

Vorzugsweise wird während der Fermentation zunächst die Aktivität des lytischen Proteins inhibiert oder die Expression des Lysegens reprimiert und erst zu einem gewünschten Zeitpunkt, vorzugsweise in der späten logarithnischen Phase, die Inhibierung oder Repression aufgehoben.

In einer weiteren bevorzugten Ausführungsform wird das so gewonnene trägergebundene, rekombinante Protein mit einem gegebenenfalls derivatisierten Bindungspartner für das Protein inkubiert und das entstandene Konjugat isoliert. Als Bindungspartner sind die oben genannten Partner der Bindungspaare geeignet.

In einer weiteren bevorzugten Ausführungsform werden die Gene von mindestens zwei verschiedenen rekombinanten Proteinen erfindungsgemäß Exprimiert. Dadurch können Immunogene oder Vakzine erhalten werden, die mehrere antigene Strukturen aufweisen. Bensonders bevorzugt ist es hierbei, als rekombinante Proteine die antigenen Determinanten von verschiedenen Viren oder Retroviren (z.B. HIV1, HIV2, HBV und EBV) zu verwenden. Zur Expression können diese Gene in einem Expressionsvektor entweder als offener Leserahmen in 3'-Richtung nach dem Gen der Targetsequenz angeordnet sein oder es kann für jedes zu exprimierende rekombinante Protein ein eigener Vektor verwendet werden. In diesem Fall ist es jedoch erforderlich, daß die Vektoren mit jeweils anderen origins of replication und anderen Resistenzgenen versehen sind.

Ein weiterer Gegenstand der Erfindung ist ein Verfahren zur Herstellung von Antikörpern, welches dadurch gekennzeichnet ist, daß ein Säugetier mit einem trägergebundenen, rekombinanten Protein, welches erhältlich ist durch Expression eines Fusionsprotein in gramnegativen Bakterien und welches mindestens eine hydrophobe, nicht lytisch wirkende membran-integrierende Proteindomäne sowie das rekombinante Protein enthält, gegebenenfalls dazu verzögerter Expression eines lytisch wirkenden Membranproteins aus Bacteriophagen oder eines lytisch wirkenden Toxin-release Gens oder lytisch wirkender Teilsequenzen davon immunisiert wird und die Antikörper aus dem Serum oder der Milz nach bekannten Verfahren gewonnen werden.

In einer bevorzugten Ausführungsform werden B-Lymphozyten der immunisierten Tiere in Gegenwart von transformierenden Agenzien mit einer geeigneten Zellinie fusioniert, die Zellinie, welche die gewünschten Antikörper produziert, kloniert und kultiviert und aus den Zellen oder dem Kulturüberstand die monoklonalen Antikörper gewonnen.

Es hat sich gezeigt, daß das erfindungsgemäße Verfahren besonders zur Herstellung von viralen Immunogenen, wie z.B. HIV-Immunogenen, HBV-Immunogenen, geeignet ist.

Ebenso hat sich überraschenderweise gezeigt, daß rekombinante Antigene, die bei der Expression in Prokaryonten üblicherweise in inaktiver Form als refractile bodies anfallen (z.B. Humanproteine wie tPA oder G-CSF) bei der Expression nach dem erfindungsgemäßen Verfahren in ihrer Aktivität und damit in ihren antigenen Strukturen erhalten bleiben. Damit erweist sich das erfindungsgemäße Verfahren als besonders vorteilhaft bei der Herstellung immunogener rekombinanter Humanproteine.

Ein weiterer Gegenstand der Erfindung ist die Verwendung der erfindungsgemäßen trägergebundenen rekombinanten Proteine als Impfstoffe (Vakzine) und zur Stimulierung von T-Lymphozyten.

Die erfindungsgemäßen Impfstoffe können in üblicher Weise hergestellt und verwendet werden.

Ein weiterer Gegenstand der Erfindung ist ein Verfahren zur Herstellung von Impfstoffen unter Verwendung der erfindungsgemäßen trägergebundenen, rekombinanten Proteine. Die Herstellung dieser Impfstoffe kann nach den bekannten Methoden durchgeführt werden. Bevorzugt wird jedoch das trägergebundene, rekombinante Protein zunächst lyophilisiert und anschließend, ggf. unter Zusatz von Hilfsstoffen, suspendiert.

Es ist weiter bevorzugt, das Vakzin als multivalentes Vakzin zu formulieren. Hierzu kann das erfindungsgemäße trägergebundene rekombinante Protein mehrere an der Membran des Bakterienghosts immobilisierte rekombinante Antigene enthalten.

Die Impfung mit dem erfindungsgemäßen Vakzin kann auf die jedem Fachmann geläufigen Methoden erfolgen, beispielsweise intradermal, intramuskular, intraperitoneal, intravenös, subkutan, oral und intranasal.

Zur intramuskulären oder subkutanen Gabe kann das Vakzin beispielsweise in physiologischer Kochsalzlösung suspendiert sein. Zur intranasalen oder intraoccularen Applikation kann das Vakzin, beispielsweise in Form eines Sprays oder einer wäßrigen Lösung angewendet werden. Für lokale, beispielsweise orale Gabe ist es häufig erforderlich, die Immunogene zeitweise gegen Inaktivierung zu schützen,beispielsweise gegen saccharolytische Enzyme in der Mundhöhle oder gegen proteolytische Enzyme im Magen. Ein derartiger vorübergehender Schutz kann beispielsweise durch Verkapselung der Immunogene erfolgen. Diese Verkapselung kann beispielsweise durch Überziehen mit einem Schutzmittel (Mikroverkapselung) oder bei Einbettung einer Vielzahl von erfindungsgemäßen Immunogenen in einen schützenden Träger (Makroverkapselung) erfolgen.

Das Verkapselungsmaterial kann semipermiabel sein oder beim Einbringen in den menschlichen oder tierischen Körper semipermiabel werden. Üblicherweise wird für die Verkapselung eine biologisch abbaubare Substanz als Träger verwendet.

Die nachfolgenden Beispiele, Abbildungen und Sequenzprotokolle erläutern die Erfindung weiter.
Fig.1 zeigt schematische Darstellungen der Plasmide pkSELS, pML1 und pMTV1
Fig.2 Schematische Darstellung eines Bakterienghosts, als Träger rekombinanter Proteine
   a) Längsschnitt durch ein gramnegatives Bakterium (om: äußere Membran; pp: periplasmatischer Raum, im: innere (cytoplasmatische) Mebran, cp: Cytoplasma).
   b) Ausbildung eines transmembranen Lysetunnels.
   c) Durch den Lysetunnes ausströmendes Cytoplasma.
   d) Bakterienghost mit Fusionsproteinen, die im Zellwandkomplex über Targetsequenzen verankert sind.

### Beispiel 1

### N-terminales Membrantargeting für HIV 1 gp41.

Aus dem Plasmid pHF14 wird ein HIV 1 spezifisches DNA-Fragment durch einen partiellen Hincll/Pvull Verdau als ein 1445bp DNA-Fragment isoliert. Das Fragment enthält die gesamte Sequenz von gp41, (345 Codons von gp41) Linker Sequenzen, die letzten 45 Codons von gp120. Es entspricht den Nucleotiden 4 bis 1448 aus SEQ ID NO: 1.

Nach Linearisieren von Plasmid pKSEL5 (SEQ ID NO:6) mit Accl und Auffüllen der überhängenden DNA-Enden mit Klenow Polymerase wird das HIV1 spezifische DNA-Fragment mit diesem linearisiertem Plasmid ligiert. Das entstandene Plasmid wird mit pHIE1 bezeichnet und enthält in frame eine Fusion einer Teilsequenz des E-Gens (E'-Targetsequenz) von PhiX174 mit dem oben genannten HIV1-Fragment, wobei das natürliche Stoppcodon des HIV1 env-Gens erhalten bleibt.

### Beispiel 2

### N- sowie C- terminales Membrantargeting von HIV1 gp41.

Aus Plasmid pHF14 wird durch Hincll-Verdau ein 1059 bp HIV1 spezifisches DNA-Fragment isoliert. Dieses Fragment enthält 5'-seitig Linker Sequenzen gefolgt von 45 Codons aus gp120 sowie 301 Codons von gp41. Es entspricht den Nucleotiden 4 bis 1062 aus SEQ ID NO:1. Nach Linearisieren von Plasmid pKSEL5 mit Accl und nach Auffüllen der überstehenden DNA-Enden mit Klenow Polymerase wurde das HIV1 spezifische DNA-Fragment mit diesem Vektor ligiert. Das entstandene Plasmid pHIE3 enthält eine in frame Fusion einerTeilsequenz des E-Gens (E'-Targetsequenz) mit einer HIV Teilsequenz und einerTeilsequenz des L-Gens ( L'-Targetsequenz).

### Beispiel 3

### C-terminales Membrantargeting von HIV1 gp41.

Aus Plasmid pHF14 wird mit Sall und Hincll ein 1061 bp DNA-Fragment isoliert. Dieses Fragment enthält 5'-seitig Linker Sequenzen gefolgt von 45 Codons gp120 sowie 301 Codons gp41. Es entspricht den Nucleotiden 2 bis 1062 aus SEQ ID NO: 1. Nach Entfernen der E'-Sequenz aus dem Plasmid pKSEL5 durch Xhol/Accl-Verdau werden mit Hilfe von Klenow Polymerase die überstehenden DNA-Enden des Vektors und des isolierten HIV1 Fragment aufgefüllt und ligiert. Das entstandene Plasmid pHIE5 enthält eine in frame Fusion der ersten 5 Codons des lacZ-Gens, Polylinker Codons, gp120/gp41 Codons und Polylinker Codons gefolgt von der L'-Targetsequenz.

### Beispiel 4

### C-terminales Membrantargeting von Streptavidin.

Das 498 bp mit Klenow Polymerase aufgefüllte Xbal-Fragment (FXaStrpA, Nucleotid 2 bis 499 von SEQ ID NO:2) aus pFN6 wird in das Plasmid pKSEL5,aus welchem das E'-Gen-Fragment durch Schnitt mit Hincll/Xhol deletiert wurde, in die aufgefüllten Schnittstellen ligiert. Das erhaltene Plasmid wird mit pAV5 bezeichnet. Damit ergibt sich im Plasmid pAV5 eine in frame Fusion der ersten 5 Codons des LacZ-Gens, 26 Aminosäurecodons aus der verbleibenden Polylinker Sequenz sowie der Aminosäuresequenzen des FXaStrpA-Anteils gefolgt von der L'Targetsequenz.

### Beispiel 5

### N-terminales Membrantargeting von Streptavidin.

Aus Plasmid pFN6 wird nach BamHI-Verdau das 5'-seitig um eine Faktor Xa-Proteasen-Schnittstelle erweitertes Streptavidin-Gen als 511 bp Fragment isoliert. Es enthält Nucleotid 14 bis 524 aus SEQ ID NO:2. Dieses DNA-Fragment wird nach Auffüllen der Enden mit Klenow Polymerase in die aufgefüllte Xbal Schnittstelle des Vektors pKSEL5 zwischen die E'und L' Targetsequenzen integriert. In Plasmid pAV1 ist damit in frame eine Gen-Fusion aus der E'-Targetsequenz und der FXaStrpA-Sequenz erfolgt. Die 3'-seitig des Streptavidins vorkommenden Stoppcodons bleiben durch die vorgenommene Klonierung erhalten.

### Beispiel 6

### N- und C- terminales Membrantargeting von Streptavidin.

Die in Plasmid pAV1 hinter dem Streptavidin-Gen vorkommenden Stoppcodons 5'-TAATAA-3'werden durch die Deletion eines 33bp großen DNA-Fragments, das durch partiellen Hincll und nachfolgenden Xbal Verdau erzeugt wird, entfernt. Die Streptavidinspezifische DNA-Sequenz enthält Nucleotid 14 bis 499 aus SEQ ID NO:2. Nach Auffüllen der Plasmid-Enden mit Klenow Polymerase und Religation, fusioniert die auf dem Vektor enthaltene L'-Targetsequenz in frame an die E'-Targetsequenz und die FXaStrpA-Sequenz (Plasmid pAV3). Das entsprechende Genprodukt verfügt damit über eine N- sowie C-terminale Targetsequenz.

### Beispiel 7

### N-terminales Membrantargeting von ß-Galactosidase.

Aus dem Plasmid pMC1403 (J. Bacteriol. 143 (1980) 971 - 980) wird mit Hilfe von Pstl und Dral ein 3124 bp DNA-Fragment (SEQ ID NO:3) isoliert und gerichtet in die Pstl und Nrul Restriktionsstellen des Plasmids pKSEL5 ligiert. Das entstandene Plasmid pLZ1 enthält die ersten 54 Codons der E'-Targetsequenz, 13 Linker-Codons und 1015 Codons des LacZ Gens. Das für Plasmid pLZ1 verwendete Pstl/Dral-Fragment erstreckt sich im Sequenzprotokoll SEQ ID NO: 3 von Nucleotid 26 bis einschließlich 3149 und umfaßt 3124bp.

### Beispiel 8

### N- und C- terminales Membrantargeting von ß-Galactosidase.

Aus Plasmid pMC1403 wird das 3010 bp LacZ DNA-Fragment (Pstl - EcoRI, Nucleotide 26 bis 3035 aus SEQ ID NO: 3) isoliert und in die Pstl/Hindlll Restriktionsstelle von pKSEL5 nach Auffüllen der EcoRI bzw. Hindlll Enden gerichtet integriert. Damit ergibt, sich in dem so erhaltenen Plasmid pLZ3 eine Fusion in frame der E'-Targetsequenz mit dem LacZ-Gen und der L'-Targetsequenz.

### Beispiel 9

### C-terminales Membrantargeting von ß-Galactosidase.

Plasmid pLZ3 wird mit EcoRI und partiell mit Accl verdaut. Dadurch wird die E'-Targetsequenz entfernt. Das Fragment enthält die Nucleotide 29 - 3035 aus SEQ ID NO:3 und ist 3007 bp lang (nach Auffüllen der EcoRI-Schnittstelle). Nach Auffüllen der überstehenden DNA-Enden des Vektors und Religation ergibt sich der Vektor pLZ5 in welchem ein lacZ-L'-Fusionsgen vorliegt und dessen Genprodukt über C-terminale Membrantargetsequenz verfügt.

### Beispiel10

### Herstellung der trägergebundenen rekombinanten Proteine über die Plasmide pMTV1 (SEQ ID NO:4), pkSEL und pML1 (SEQ ID NO:5).

Auf den Plasmiden pMTV1 und pML1 befindet sich eine Lysekassette, bestehend aus dem Lambda c1857 Repressor-Gen, dem rechtsseitigen Lambdapromotor/Operator System pR sowie dem PhiX174 Lysegen E. Die Integration des Fremdgens kann in der multiple cloning site mcs 2 für pMTV1 oder pkSEL5 (Fig.1) erfolgen. Dabei wird in analoger Weise wie in den Beispielen 1 - 9 beschrieben vorgegangen.

### Beispiel 11

### Fermentation und Lyse

Das Plasmid wird in E. coli K12 (DSM 2093) integriert und die Kultur im Schüttelkolben bis zu OD 0,8 - 1,2 bei 600 nm angezogen, wobeidie Expression des Lysegens E durch c1857 Repressormoleküle reprimiert ist(Eur. J. Biochem. 180 (1989) 393 bis 398). Durch Temperaturerhöhung auf 42°C während der exponentiellen Wachstumsphase der Bakterien erfolgt die Expression von Gen-E durch thermische Inaktivierung der c1857 Repressormoleküle. Die durch Protein E verursachte Lyse von E. coli setzt je nach Kulturmedium (Voll- oder Minimalmedium, unter Belüftung im Schüttelwasserbad) der Bakterien zwischen 10 bis 30 min nach Temperaturerhöhung ein. Nach weiteren 10 bis 30 min ist die Lyse vollständig.

### Beispiel 12

### Modifizierte Protein E-Lyse.

Es wird wie in Beispiel 11 kultiviert, wobei jedoch 30 min. vor Temperaturerhöhung von 28°C auf 42°C das Kulturmedium durch Zugabe von Magnesiumsulfatlösung auf 0,2 mol/I Magnesiumsulfat gebracht wird. Dies verhindert trotz Expression von Gen E die Lyse der Bakterien.

30 min. nach Temperaturerhöhung werden die Zellen durch Zentrifugation geerntet. Durch Resuspension des Zellpellets in niedermolarem Puffer (PBS, 1 mmol/1 Phosphatpuffer, 1 bis 10 mmol/I Tris - HCI pH 6 - 8) oder Wasser erfolgt eine sofortige Lyse der Zellen. Die dabei anfallenden Zellhüllen werden als Bakterienghosts bezeichnet. Bei diesen Bedingungen, die einer Kombination von Protein E-Lyse und osmotischem Schock entsprechen, wird eine größere Lysestruktur in den Bakterien erreicht. Die Morphologie der Bakterienghots bleibt auch unter diesen Bedingungen weitgehend erhalten.

Zur Reinigung werden die Bakterienghost 2 x mit PBS oder 0,9 % NaCI gewaschen (resuspendieren und zentrifugieren) und gefriergetrocknet.

### Beispiel 13

### Immunisierung

10⁹ Keime (ensprechend 1 mg Bakterienghosts Trockengewicht) pro Maus werden in 0,9 % NaCI intraperitonal 4 x in monatlichen Abständen zur Immunisierung gegeben. 8 Tage nach der letzten Immunisierung wird Serum gewonnen und die Antikörper werden isoliert.

### Beispiel 14

### Bindung von biotinyliertem HBc-Antigen

Nach Beispiel 4 hergestellte Bakterienghosts, in die Streptavidin über Targetsequenzen integriert ist, werden lyophilisiert. 1 mg dieses Lyophilisats wird 10 ml einer Lösung von 20 Ig/ml eines Konjugats aus Hepatitis B core-Antigen und Biotin (hergestellt durch Reaktion von HBcAg mit N-Hydroxysuccinimid-aktiviertem Biotin) in 40 mmol/1 Phosphatpuffer, pH 7,4, 30 min inkubiert und anschließend mehrfach mit 40 mmol/1 Phosphatpuffer, pH 7,4, gewaschen. Auf diese Weise wird ein trägergebundenes HBcAg-Immunogen erhalten, das zur Immunsierung und Gewinnung von Antikörpern verwendet werden kann.

Sequenzprotokolle

SEQ ID NO:1
ART DER SEQUENZ:Nucleotidsequenz     HIV1(gp120/gp41)
SEQUENZLÄNGE:1451 Basenpaare

STRANGFORM:Einzelstrang

gtcgacctgc aggcatgcaa gctGATCTTC AGACCTGGAG GAGGAGATAT GAGGGACAAT 60
TGGAGAAGTG AATTATATAA ATATAAAGTA GTAAAAATTG AACCATTAGG AGTAGCACCC 120
ACCAAGGCAA AGAGAAGAGT GGTGCAGAGA GAAAAAAGAG CAGTGGGAAT AGGAGCTTTG 180
TTCCTTGGGT TCTTGGGAGC AGCAGGAAGC ACTATGGGCG CAGCGTCAAT GACGCTGACG 240
GTACAGGCCA GACAATTATT GTCTGGTATA GTGCAGCAGC AGAACAATTT GCTGAGGGCT 300
ATTGAGGGCC AACAGCATCT GTTGCAACTC ACAGTCTGGG GCATCAAGCA GCTCCAGGCA 360
AGAATCCTGG CTGTGGAAAG ATACCTAAAG GATCAACAGC TCCTGGGGAT TTGGGGTTGC 420
TCTGGAAAAC TCATTTGCAC CACTGCTGTG CCTTGGAATG CTAGTTGGAG TAATAAATCT 480
CTGGAACAGA TTTGGAATAA CATGACCTGG ATGGAGTGGG ACAGAGAAAT TAACAATTAC 540
ACAAGCTTAA TACACTCCTT AATTGAAGAA TCGCAAAACC AGCAAGAAAA GAATGAACAA 600
GAATTATTGG AATTAGATAA ATGGGCAAGT TTGTGGAATT GGTTTAACAT AACAAATTGG 660
CTGTGGTATA TAAAATTATT CATAATGATA GTAGGAGGCT TGGTAGGTTT AAGAATAGTT 720
TTTGCTGTAC TTTCTATAGT GAATAGAGTT AGGCAGGGAT ATTCACCATT ATCGTTTCAG 780
ACCCACCTCC CAAACCCGAG GGGACCCGAC AGGCCCGAAG GAATAGAAGA AGAAGGTGGA 840
GAGAGAGACA GAGACAGATC CATTCGATTA GTGAACGGAT CCTTAGCACT TATCTGGGAC 900
GATCTGCGGA GCCTGTGCCT CTTCAGCTAC CACCGCTTGA GAGACTTACT CTTGATTGTA 960
ACGAGGATTG TGGAACTTCT GGGACGCAGG GGGTGGGAAG CCCTCAAATA TTGGTGGAAT 1020
CTCCTACAGT ATTGGAGTCA GGAACTAAAG AATAGTGCTG TTAACTTGCT CAATGCCACA 1080
GCTATAGCAG TAGCTGAGGG GACAGATAGG GTTATAGAAT TAGTACAAGC AGCTTATAGA 1140
GCCATTCGCC ACATACCTAG AAGAATAAGA CAGGGCTTGG AAAGGATTTT GCTATAAgat 1200
gggtggcaag tggtcaaaaa gtagtgtggt tggatggcct gctgtaaggg aaagaatgag 1260
acgagctgag ccagcagcag atggggtggg agcagtatct cgagacctag aaaaacatgg 1320
agcaatcaca agtagcaata cagcagctac caatgccgat tgtgcttggc tagaagcaca 1380
agaggaggag gaggtgggtt ttccagtcac acctcaggta cctttaagac caatgactta 1440
caaggcagct g 1451

In Großbuchstaben dargestellt ist der C-Terminus von gp160 von HIV1
(Originalkoordinaten des BH8-Klons: 7199 bis 8372 (nach Ratner et al.
1985)). Nach den 45 letzten Codons des C-Terminus von gp120 (5'-
ATCTTCAGA ......GAAAAAAGA-3') folgen die 345 Codons des gp41 ( 5'-
GCAGTGGGAA.....TTTTGCTATAA-3'). 5'-seitig der HIV1-Sequenz erstreckt
sich der für die folgenden Klonierungen verwendete Polylinker.

Referenz: Ratner, L., Haseltine, W., Patarca, R., Livak, K.J., Starich,
B., Josephs, S.F., Doran, E.R., Rafalski, J.A., Whitehorn, E.A.,
Baumeister, K., Ivanoff, L., Petteway, Jr. S.R., Pearson, M.L.,
Lautenberger, J.A., Papas, T.S., Ghrayeb, J., Chang, N.T., Gallo, R.C.,
Wong-Staal, F. 1985. Complete nucleotide sequence of the AIDS virus,
HTLV-III. Nature 313: 277 - 284.

SEQ ID NO:2
ART DER SEQUENZ:Nucleotidsequenz     (FXa-Strpa)
SEQUENZLÄNGE:525 Basenpaare

STRANGFORM:Einzelstrang


DNA sequence  FXa-StrpA    525  b.p. complete sequence ;

tctagaacta gtggatccat cgagggtagg tctATGGACC CGTCCAAGGA CTCCAAAGCT  60
CAGGTTTCTG CAGCCGAAGC TGGTATCACT GGCACCTGGT ATAACCAACT GGGGTCGACT 120
TTCATTGTGA CCGCTGGTGC GGACGGAGCT CTGACTGGCA CCTACGAATC TGCGGTTGGT 180
AACGCAGAAT CCCGCTACGT ACTGACTGGC CGTTATGACT CTGCACCTGC CACCGATGGC 240
TCTGGTACCG CTCTGGGCTG GACTGTGGCT TGGAAAAACA ACTATCGTAA TGCGCACAGC 300
GCCACTACGT GGTCTGGCCA ATACGTTGGC GGTGCTGAGG CTCGTATCAA CACTCAGTGG 360
CTGTTAACAT CCGGCACTAC CGAAGCGAAT GCATGGAAAT CGACACTAGT AGGTCATGAC 420
ACCTTTACCA AAGTTAAGCC TTCTGCTGCT AGCATTGATG CTGCCAAGAA AGCAGGCGTA 480
AACAACGGTA ACCCTCTAGA CGCTGTTCAG CAATAAtaag gatcc                525

In Großbuchstaben dargestellt ist die Streptavidinsequenz (Argarana et
al. 1986). Die für die Faktor Xa-Spaltstelle Ile-Glu-Gly-Arg  kodierende
DNA-Sequenz 5'-atcgagggtagg-3' reicht in der hier aufgeführten Sequenz
von Nukleotid 19 bis 30.

Referenz :

Argarana, C.E., Kuntz, I.D., Birken, S., Axel, R., Cantor, Ch.R. 1986.
Molecular cloning and nucleotide sequence of the streptavidin gene.
Nucl. Acids Res. 14 (4): 1871 - 1882.

SEQ ID NO:3
ART DER SEQUENZ:Nucleotidsequenz (LacZ)
SEQUENZLÄNGE:3152 Basenpaare

STRANGFORM:Einzelstrang

ATGACCATGA TTACGAATTG CTGCAGGTCG ACGGATCCCG TCGTTTTACA ACGTCGTGAC 60
TGGGAAAACC CTGGCGTTAC CCAACTTAAT CGCCTTGCAG CACATCCCCC TTTCGCCAGC 120
TGGCGTAATA GCGAAGAGGC CCGCACCGAT CGCCCTTCCC AACAGTTGCG CAGCCTGAAT 180
GGCGAATGGC GCTTTGCCTG GTTTCCGGCA CCAGAAGCGG TGCCGGAAAG CTGGCTGGAG 240
TGCGATCTTC CTGAGGCCGA TACTGTCGTC GTCCCCTCAA ACTGGCAGAT GCACGGTTAC 300
GATGCGCCCA TCTACACCAA CGTAACCTAT CCCATTACGG TCAATCCGCC GTTTGTTCCC 360
ACGGAGAATC CGACGGGTTG TTACTCGCTC ACATTTAATG TTGATGAAAG CTGGCTACAG 420
GAAGGCCAGA CGCGAATTAT TTTTGATGGC GTTAACTCGG CGTTTCATCT GTGGTGCAAC 480
GGGCGCTGGG TCGGTTACGG CCAGGACAGT CGTTTGCCGT CTGAATTTGA CCTGAGCGCA 540
TTTTTACGCG CCGGAGAAAA CCGCCTCGCG GTGATGGTGC TGCGTTGGAG TGACGGCAGT 600
TATCTGGAAG ATCAGGATAT GTGGCGGATG AGCGGCATTT TCCGTGACGT CTCGTTGCTG 660
CATAAACCGA CTACACAAAT CAGCGATTTC CATGTTGCCA CTCGCTTTAA TGATGATTTC 720
AGCCGCGCTG TACTGGAGGC TGAAGTTCAG ATGTGCGGCG AGTTGCGTGA CTACCTACGG 780
GTAACAGTTT CTTTATGGCA GGGTGAAACG CAGGTCGCCA GCGGCACCGC GCCTTTCGGC 840
GGTGAAATTA TCGATGAGCG TGGTGGTTAT GCCGATCGCG TCACACTACG TCTGAACGTC 900
GAAAACCCGA AACTGTGGAG CGCCGAAATC CCGAATCTCT ATCGTGCGGT GGTTGAACTG 960
CACACCGCCG ACGGCACGCT GATTGAAGCA GAAGCCTGCG ATGTCGGTTT CCGCGAGGTG 1020
CGGATTGAAA ATGGTCTGCT GCTGCTGAAC GGCAAGCCGT TGCTGATTCG AGGCGTTAAC 1080
CGTCACGAGC ATCATCCTCT GCATGGTCAG GTCATGGATG AGCAGACGAT GGTGCAGGAT 1140
ATCCTGCTGA TGAAGCAGAA CAACTTTAAC GCCGTGCGCT GTTCGCATTA TCCGAACCAT 1200
CCGCTGTGGT ACACGCTGTG CGACCGCTAC GGCCTGTATG TGGTGGATGA AGCCAATATT 1260
GAAACCCACG GCATGGTGCC AATGAATCGT CTGACCGATG ATCCGCGCTG GCTACCGGCG 1320
ATGAGCGAAC GCGTAACGCG AATGGTGCAG CGCGATCGTA ATCACCCGAG TGTGATCATC 1380
TGGTCGCTGG GGAATGAATC AGGCCACGGC GCTAATCACG ACGCGCTGTA TCGCTGGATC 1440
AAATCTGTCG ATCCTTCCCG CCCGGTGCAG TATGAAGGCG GCGGAGCCGA CACCACGGCC 1500
ACCGATATTA TTTGCCCGAT GTACGCGCGC GTGGATGAAG ACCAGCCCTT CCCGGCTGTG 1560
CCGAAATGGT CCATCAAAAA ATGGCTTTCG CTACCTGGAG AGACGCGCCC GCTGATCCTT 1620
TGCGAATACG CCCACGCGAT GGGTAACAGT CTTGGCGGTT TCGCTAAATA CTGGCAGGCG 1680
TTTCGTCAGT ATCCCCGTTT ACAGGGCGGC TTCGTCTGGG ACTGGGTGGA TCAGTCGCTG 1740
ATTAAATATG ATGAAAACGG CAACCCGTGG TCGGCTTACG GCGGTGATTT TGGCGATACG 1800
CCGAACGATC GCCAGTTCTG TATGAACGGT CTGGTCTTTG CCGACCGCAC GCCGCATCCA 1860
GCGCTGACGG AAGCAAAACA CCAGCAGCAG TTTTTCCAGT TCCGTTTATC CGGGCAAACC 1920
ATCGAAGTGA CCAGCGAATA CCTGTTCCGT CATAGCGATA ACGAGCTCCT GCACTGGATG 1980
GTGGCGCTGG ATGGTAAGCC GCTGGCAAGC GGTGAAGTGC CTCTGGATGT CGCTCCACAA 2040
GGTAAACAGT TGATTGAACT GCCTGAACTA CCGCAGCCGG AGAGCGCCGG GCAACTCTGG 2100
CTCACAGTAC GCGTAGTGCA ACCGAACGCG ACCGCATGGT CAGAAGCCGG GCACATCAGC 2160
GCCTGGCAGC AGTGGCGTCT GGCGGAAAAC CTCAGTGTGA CGCTCCCCGC CGCGTCCCAC 2220
GCCATCCCGC ATCTGACCAC CAGCGAAATG GATTTTTGCA TCGAGCTGGG TAATAAGCGT 2280
TGGCAATTTA ACCGCCAGTC AGGCTTTCTT TCACAGATGT GGATTGGCGA TAAAAAACAA 2340
CTGCTGACGC CGCTGCGCGA TCAGTTCACC CGTGCACCGC TGGATAACGA CATTGGCGTA 2400
AGTGAAGCGA CCCGCATTGA CCCTAACGCC TGGGTCGAAC GCTGGAAGGC GGCGGGCCAT 2460
TACCAGGCCG AAGCAGCGTT GTTGCAGTGC ACGGCAGATA CACTTGCTGA TGCGGTGCTG 2520
ATTACGACCG CTCACGCGTG GCAGCATCAG GGGAAAACCT TATTTATCAG CCGGAAAACC 2580
TACCGGATTG ATGGTAGTGG TCAAATGGCG ATTACCGTTG ATGTTGAAGT GGCGAGCGAT 2640
ACACCGCATC CGGCGCGGAT TGGCCTGAAC TGCCAGCTGG CGCAGGTAGC AGAGCGGGTA 2700
AACTGGCTCG GATTAGGGCC GCAAGAAAAC TATCCCGACC GCCTTACTGC CGCCTGTTTT 2760

GACCGCTGGG ATCTGCCATT GTCAGACATG TATACCCCGT ACGTCTTCCC GAGCGAAAAC 2820
GGTCTGCGCT GCGGGACGCG CGAATTGAAT TATGGCCCAC ACCAGTGGCG CGGCGACTTC 2880
CAGTTCAACA TCAGCCGCTA CAGTCAACAG CAACTGATGG AAACCAGCCA TCGCCATCTG 2940
CTGCACGCGG AAGAAGGCAC ATGGCTGAAT ATCGACGGTT TCCATATGGG GATTGGTGGC 3000
GACGACTCCT GGAGCCCGTC AGTATCGGCG GAATTCCAGC TGAGCGCCGG TCGCTACCAT 3060
TACCAGTTGG TCTGGTGTCA AAAATAAtaa taaccgggca ggccatgtct gcccgtattt 3120
cgcgtaagga aatccattat gtactattta aa 3152

In Großbuchstaben ist die DNA-Sequenz für das LacZ-Gen aus Plasmid pMC
1403 dargestellt.

Referenz : Casadaban, M.J., Chou, J., Cohen, M.S. 1980. In vitro gene
fusions that join an enzymatically active ß-galactosidase segment to
amino-terminal fragments of exogenous proteins: Escherichia coli plasmid
vectors for the detection and cloning of translational initiation
signals. J. Bacteriol. 143: 971 - 980.

SEQ ID NO:4
ART DER SEQUENZ:Nucleotidsequenz (pMTV1)
SEQUENZLÄNGE:5314 Basenpaare

STRANGFORM:Einzelstrang

AAATTGTAAA CGTTAATATT AGACATAATT TATCCTCAAG TAAGGGGCCG AAGCCCCTGC 60
AATTAAAATT GTTGACCACC TACATACCAA AGACGAGCGC CTTTACGCTT GCCTTTAGTA 120
CCTCGCAACG GCTGCGGACG ACCAGGGCGA GCGCCAGAAC GTTTTTTACC TTTAGACATT 180
ACATCACTCC TTCCGCACGT AATTTTTGAC GCACGTTTTC TTCTGCGTCA GTAAGAACGT 240
CAGTGTTTCC TGCGCGTACA CGCAAGGTAA ACGCGAACAA TTCAGCGGCT TTAACCGGAC 300
GCTCGACGCC ATTAATAATG TTTTCCGTAA ATTCAGCGCC TTCCATGATG AGACAGGCCG 360
TTTGAATGTT GACGGGATGA ACATAATAAG CAATGACGGC AGCAATAAAC TCAACAGGAG 420
CAGGAAAGCG AGGGTATCCC ACAAAGTCCA GCGTACCATA AACGCAAGCC TCAACGCAGC 480
GACGAGCACG AGAGCGGTCA GTAGCAATCC AAACTTTGTT ACTCGTCAGA AAATCGAAAT 540
CATCTTCGGT TAAATCCAAA ACGGCAGAAG CCTGAATTCT AGCTAGAGGA TCTTTAGCTG 600
TCTTGGTTTG CCCAAAGCGC ATTGCATAAT CTTTCAGGGT TATGCGTTGT TCCATACAAC 660
CTCCTTAGTA CATGCAACCA TTATCACCGC CAGAGGTAAA ATAGTCAACA CGCACGGTGT 720
TAGATATTTA TCCCTTGCGG TGATAGATTT AACGTATGAG CACAAAAAAG AAACCATTAA 780
CACAAGAGCA GCTTGAGGAC GCACGTCGCC TTAAAGCAAT TTATGAAAAA AAGAAAAATG 840
AACTTGGCTT ATCCCAGGAA TCTGTCGCAG ACAAGATGGG GATGGGGCAG TCAGGCGTTG 900
GTGCTTTATT TAATGGCATC AATGCATTAA ATGCTTATAA CGCCGCATTG CTTACAAAAA 960
TTCTCAAAGT TAGCGTTGAA GAATTTAGCC CTTCAATCGC CAGAGAAATC TACGAGATGT 1020
ATGAAGCGGT TAGTATGCAG CCGTCACTTA GAAGTGAGTA TGAGTACCCT GTTTTTTCTC 1080
ATGTTCAGGC AGGGATGTTC TCACCTAAGC TTAGAACCTT TACCAAAGGT GATGCGGAGA 1140
GATGGGTAAG CACAACCAAA AAAGCCAGTG ATTCTGCATT CTGGCTTGAG GTTGAAGGTA 1200
ATTCCATGAC CGCACCAACA GGCTCCAAGC CAAGCTTTCC TGACGGAATG TTAATTCTCG 1260
TTGACCCTGA GCAGGCTGTT GAGCCAGGTG ATTTCTGCAT AGCCAGACTT GGGGGTGATG 1320
AGTTTACCTT CAAGAAACTG ATCAGGGATA GCGGTCAGGT GTTTTTACAA CCACTAAACC 1380
CACAGTACCC AATGATCCCA TGCAATGAGA GTTGTTCCGT TGTGGGGAAA GTTATCGCTA 1440
GTCAGTGGCC TGAAGAGACG TTTGGCTGAT CGGCAAGGTG TTCTGGTCGG CGCATAGCTG 1500
ATAACAATTG AGCAAGAATC TTCATCGAAT TAGGGGAATT TTCACTCCCC TCAGAACATA 1560
ACATAGTAAA TGGATTGAAT TATGAAGAAT GGTTTTTATG CGACTTACCG CAGCAAAAAT 1620
AAAGGGAAAG ATACTTGAAG ACGAAAGGGC ATTTTGTTAA AATTCGCGTT AAATTTTTGT 1680
TAAATCAGCT CATTTTTTAA CCAATAGGCC GAAATCGGCA AAATCCCTTA TAAATCAAAA 1740
GAATAGACCG AGATAGGGTT GAGTGTTGTT CCAGTTTGGA ACAAGAGTCC ACTATTAAAG 1800
AACGTGGACT CCAACGTCAA AGGGCGAAAA ACCGTCTATC AGGGCGATGG CCCACTACGT 1860
GAACCATCAC CCTAATCAAG TTTTTTGGGG TCGAGGTGCC GTAAAGCACT AAATCGGAAC 1920
CCTAAAGGGA GCCCCCGATT TAGAGCTTGA CGGGGAAAGC CGGCGAACGT GGCGAGAAAG 1980
GAAGGGAAGA AAGCGAAAGG AGCGGGCGCT AGGGCGCTGG CAAGTGTAGC GGTCACGCTG 2040
CGCGTAACCA CCACACCCGC CGCGCTTAAT GCGCCGCTAC AGGGCGCGTC CCATTCGCCA 2100
TTCAGGCTAC GCAACTGTTG GGAAGGGCGA TCGGTGCGGG CCTCTTCGCT ATTACGCCAG 2160
CTGGCGAAGG GGGGATGTGC TGCAAGGCGA TTAAGTTGGG TAACGCCAGG GTTTTCCCAG 2220
TCACGACGTT GTAAAACGAC GGCCAGTGAA TTGTAATACG ACTCACTATA GGGCGAATTG 2280
GAGCTCCACC GCGGTGGCGG CCGCTCTAGT ATGGTGCACT CTCAGTACAA TCTGCTCTGA 2340
TGCCGCATAG TTAAGCCAGT ATATACACTC CGCTATCGCT ACGTGACTGG GTCATGGCTG 2400
CGCCCCGACA CCCGCCAACA CCCGCTGACG CGCCCTGACG GGCTTGTCTG CTCCCGGCAT 2460
CCGCTTACAG ACAAGCTGTG ACCGTCTCCG GGAGCTGCAT GTGTCAGAGG TTTTCACCGT 2520
CATCACCGAA ACGCGCGAGG CAGTAAGGTC GGATGCTTTG TGAGCAATTC GTCCCTTAAG 2580
TAAGCAATTG CTGTAAAGTC GTCACTGTGC GGATCACCGC TTCCAGTAGC GACAGAAGCA 2640
ATTGATTGGT AAATTTCGAG AGAAAGATCG CGAGGAAGAT CAATACATAA AGAGTTGAAC 2700
TTCTTTGTTG TCTTCGACAT GGGTAATCCT CATGTTTGAA TGGCCCTAGA GGATCCGGCC 2760
AAGCTTGCAT GCCTGCAGGT CGACTCTAGA GGATCCCCGA CGCTCGACGC CATTAATAAT 2820
GTTTTCCGTA AATTCAGCGC CTTCCATGAT GAGACAGGCC GTTTGAATGT TGACGGGATG 2880

AACATAATAA GCAATGACGG CAGCAATAAA CTCAACAGGA GCAGGAAAGC GAGGGTATCC 2940
CACAAAGTCC AGCGTACCAT AAACGCAAGC CTCAACGCAG CGACGAGCAC GAGAGCGGTC 3000
AGTAGCAATC CAAACTTTGT TACTCGTCAG AAAATCGAAA TCATCTTCGG TTAAATCCAA 3060
AACGGCAGAA GCCTGAATGA GAATTCGACC TCGAGGGGGG GCCCGGTACC CAGCTTTTGT 3120
TCCCTTTAGT GAGGGTTAAT TCCGAGCTTG GCGTAATCAT GGTCATAGCT GTTTCCTGTG 3180
TGAAATTGTT ATCCGCTCAC AATTCCACAC AACATAGGAG CCGGAAGCAT AAAGTGTAAA 3240
GCCTGGGGTG CCTAATGAGT GAGGTAACTC ACATTAATTG CGTTGCGCTC ACTGCCCGCT 3300
TTCCAGTCGG GAAACCTGTC GTGCCAGCTG CATTAATGAA TCGGCCAACG CGCGGGGAGA 3360
GGCGGTTTGC GTATTGGGCG CTCTTCCGCT TCCTCGCTCA CTGACTCGCT GCGCTCGGTC 3420
GTTCGGCTGC GGCGAGCGGT ATCAGCTCAC TCAAAGGCGG TAATACGGTT ATCCACAGAA 3480
TCAGGGGATA ACGCAGGAAA GAACATGTGA GCAAAAGGCC AGCAAAAGGC CAGGAACCGT 3540
AAAAAGGCCG CGTTGCTGGC GTTTTTCCAT AGGCTCGGCC CCCCTGACGA GCATCACAAA 3600
AATCGACGCT CAAGTCAGAG GTGGCGAAAC CCGACAGGAC TATAAAGATA CCAGGCGTTC 3660
CCCCCTGGAA GCTCCCTCGT GCGCTCTCCT GTTCCGACCC TGCCGCTTAC CGGATACCTG 3720
TCCGCCTTTC TCCCTTCGGG AAGCGTGGCG CTTTCTCAAT GCTCACGCTG TAGGTATCTC 3780
AGTTCGGTGT AGGTCGTTCG CTCCAAGCTG GGCTGTGTGC ACGAACCCCC CGTTCAGCCC 3840
GACCGCTGCG CCTTATCCGG TAACTATCGT CTTGAGTCCA ACCCGGTAAG ACACGACTTA 3900
TCGCCACTGG CAGCAGCCAC TGGTAACAGG ATTAGCAGAG CGAGGTATGT AGGCGGTGCT 3960
ACAGAGTTCT TGAAGTGGTG GCCTAACTAC GGCTACACTA GAAGGACAGT ATTTGGTATC 4020
TGCGCTCTGC TGAAGCCAGT TACCTTCGGA AAAAGAGTTG GTAGCTCTTG ATCCGGCAAA 4080
CAAACCACCG CTGGTAGCGG TGGTTTTTTT GTTTGCAAGC AGCAGATTAC GCGCAGAAAA 4140
AAAGGATCTC AAGAAGATCC TTTGATCTTT TCTACGGGGT CTGACGCTCA GTGGAACGAA 4200
AACTCACGTT AAGGGATTTT GGTCATGAGA TTATCAAAAA GGATCTTCAC CTAGATCCTT 4260
TTAAATTAAA AATGAAGTTT TAAATCAATC TAAAGTATAT ATGAGTAAAC TTGGTCTGAC 4320
AGTTACCAAT GCTTAATCAG TGAGGCACCT ATCTCAGCGA TCTGTCTATT TCGTTCATCC 4380
ATAGTTGCCT GACTGCCCGT CGTGTAGATA ACTACGATAC GGGAGGGCTT ACCATCTGGC 4440
CCCAGTGCTG CAATGATACC GCGAGACCCA CGCTCACCGG CTCCAGATTT ATCAGCAATA 4500
AACCAGCCAG CCGGAAGGGC CGAGCGCAGA AGTGGTCCTG CAACTTTATC CGCCTCCATC 4560
CAGTCTATTA ATTGTTGCCG GGAAGCTAGA GTAAGTAGTT CGCCAGTTAA TAGTTTGCGC 4620
AACGTTGTTG CCATTGCTAC AGGCATCGTG GTGTCACGCT CGTCGTTTGG TATGGCTTCA 4680
TTCAGCTCCG GTTCCCAACG ATCAAGGCGA GTTACATGAT CCCCCATGTT GTGAAAAAAA 4740
GCGGTTAGCT CCTTCGGTCC TCCGATCGTT GTCAGAAGTA AGTTGGCCGC AGTGTTATCA 4800
CTCATGCTTA TGGCAGCACT GCATAATTCT CTTACTGTCA TGCCATCCGT AAGATGCTTT 4860
TCTGTGACTG GTGAGTACTC AACCAAGTCA TTCTGAGAAT AGTGTATGCG GCGACCGAGT 4920
TGCTCTTGCC CGGCGTCAAT ACGGGATAAT ACCGCGCCAC ATAGCAGAAC TTTAAAAGTG 4980
CTCATCATTG GAAAACGTTC TTCGGGGCGA AAACTCTCAA GGATCTTACC GCTGTTGAGA 5040
TCCAGTTCGA TGTAACCCAC TCGTGCACCC AACTGATCTT CAGCATCTTT TACTTTCACC 5100
AGCGTTTCTG GGTGAGCAAA AACAGGAAGG CAAAATGCCG CAAAAAAGGG AATAAGGGCG 5160
ACACGGAAAT GTTGAATACT CATACTCTTC CTTTTTCAAT ATTATTGAAG CATTTATCAG 5220
GGTTATTGTC TCATGAGCGG ATACATATTT GAATGTATTT AGAAAAATAA ACAAATAGGG 5280
GTTCCGCGCA CATTTCCCCG AAAAGTGCCA CCTG 5314

SEQ ID NO:5
ART DER SEQUENZ:Nucleotidsequenz (pML1)
SEQUENZLÄNGE:7641 Basenpaare

STRANGFORM:Einzelstrang

GACGCCGGGC AAGAGCAACT CGGTCGCCGC ATACACTATT CTCAGAATGA CTTGGTTGAG 60
TACTCACCAG TCACAGAAAA GCATCTTACG GATGGCATGA CAGTAAGAGA ATTATGCAGT 120
GCTGCCATAA CCATGAGTGA TAACACTGCG GCCAACTTAC TTCTGACAAC GATCGGAGGA 180
CCGAAGGAGC TAACCGCTTT TTTGCACAAC ATGGGGGATC ATGTAACTCG CCTTGATCGT 240
TGGGAACCGG AGCTGAATGA AGCCATACCA AACGACGAGC GTGACACCAC GATGCCTGCA 300
GCAATGGCAA CAACGTTGCG CAAACTATTA ACTGGCGAAC TACTTACTCT AGCTTCCCGG 360
CAACAATTAA TAGACTGGAT GGAGGCGGAT AAAGTTGCAG GACCACTTCT GCGCTCGGCC 420
CTTCCGGCTG GCTGGTTTAT TGCTGATAAA TCTGGAGCCG GTGAGCGTGG GTCTCGCGGT 480
ATCATTGCAG CACTGGGGCC AGATGGTAAG CCCTCCCGTA TCGTAGTTAT CTACACGACG 540
GGGAGTCAGG CAACTATGGA TGAACGAAAT AGACAGATCG CTGAGATAGG TGCCTCACTG 600
ATTAAGCATT GGTAACTGTC AGACCAAGTT TACTCATATA TACTTTAGAT TGATTTAAAA 660
CTTCATTTTT AATTTAAAAG GATCTAGGTG AAGATCCTTT TTGATAATCT CATGACCAAA 720
ATCCCTTAAC GTGAGTTTTC GTTCCACTGA GCGTCAGACC CCTTAATAAG ATGATCTTCT 780
TGAGATCGTT TTGGTCTGCG CGTAATCTCT TGCTCTGAAA ACGAAAAAAC CGCCTTGCAG 840
GGCGGTTTTT CGAAGGTTCT CTGAGCTACC AACTCTTTGA ACCGAGGTAA CTGGCTTGGA 900
GGAGCGCAGT CACCAAAACT TGTCCTTTCA GTTTAGCCTT AACCGGCGCA TGACTTCAAG 960
ACTAACTCCT CTAAATCAAT TACCAGTGGC TGCTGCCAGT GGTGCTTTTG CATGTCTTTC 1020
CGGGTTGGAC TCAAGACGAT AGTTACCGGA TAAGGCGCAG CGGTCGGACT GAACGGGGGG 1080
TTCGTGCATA CAGTCCAGCT TGGAGCGAAC TGCCTACCCG GAACTGAGTG TCAGGCGTGG 1140
AATGAGACAA ACGCGGCCAT AACAGCGGAA TGACACCGGT AAACCGAAAG GCAGGAACAG 1200
GAGAGCGCAC GAGGGAGCCG CCAGGGGAAA CGCCTGGTAT CTTTATAGTC CTGTCGGGTT 1260
TCGCCACCAC TGATTTGAGC GTCAGATTTC GTGATGCTTG TCAGGGGGGC GGAGCCTATG 1320
GAAAAACGGC TTTGCCGCGG CCCTCTCACT TCCCTGTTAA GTATCTTCCT GGCATCTTCC 1380
AGGAAATCTC CGCCCCGTTC GTAAGCCATT TCCGCTCGCC GCAGTCGAAC GACCGAGCGT 1440
AGCGAGTCAG TGAGCGAGGA AGCGGAATAT ATCCTGTATC ACATATTCTG CTGACGCACC 1500
GGTGCAGCCT TTTTTCTCCT GCCACATGAA GCACTTCACT GACACCCTCA TCAGTGCCAA 1560
CATAGTAAGC CAGTATACAC TCCGCTAGCG CTGAGGTCTG CCTCGTGAAG AAGGTGTTGC 1620
TGACTCATAC CAGGCCTGAA TCGCCCCATC ATCCAGCCAG AAAGTGAGGG AGCCACGGTT 1680
GATGAGAGCT TTGTTGTAGG TGGACCAGTT GGTGATTTTG AACTTTTGCT TTGCCACGGA 1740
ACGGTCTGCG TTGTCGGGAA GATGCGTGAT CTGATCCTTC AACTCAGCAA AAGTTCGATT 1800
TATTCAACAA AGCCACGTTG TGTCTCAAAA TCTCTGATGT TACATTGCAC AAGATAAAAA 1860
TATATCATCA TGAACAATAA AACTGTCTGC TTACATAAAC AGTAATACAA GGGGTGTTAT 1920
GAGCCATATT CAACGGGAAA CGTCTTGCTC GAGGCCGCGA TTAAATTCCA ACATGGATGC 1980
TGATTTATAT GGGTATAAAT GGGCTCGCGA TAATGTCGGG CAATCAGGTG CGACAATCTA 2040
TCGATTGTAT GGGAAGCCCG ATGCGCCAGA GTTGTTTCTG AAACATGGCA AAGGTAGCGT 2100
TGCCAATGAT GTTACAGATG AGATGGTCAG ACTAAACTGG CTGACGGAAT TTATGCCTCT 2160
TCCGACCATC AAGCATTTTA TCCGTACTCC TGATGATGCA TGGTTACTCA CCACTGCGAT 2220
CCCCGGGAAA ACAGCATTCC AGGTATTAGA AGAATATCCT GATTCAGGTG AAAATATTGT 2280
TGATGCGCTG GCAGTGTTCC TGCGCCGGTT GCATTCGATT CCTGTTTGTA ATTGTCCTTT 2340
TAACAGCGAT CGCGTATTTC GTCTCGCTCA GGCGCAATCA CGAATGAATA ACGGTTTGGT 2400
TGATGCGAGT GATTTTGATG ACGAGCGTAA TGGCTGGCCT GTTGAACAAG TCTGGAAAGA 2460
AATGCATAAG CTTTTGCCAT TCTCACCGGA TTCAGTCGTC ACTCATGGTG ATTTCTCACT 2520
TGATAACCTT ATTTTTGACG AGGGGAAATT AATAGGTTGT ATTGATGTTG GACGAGTCGG 2580
AATCGCAGAC CGATACCAGG ATCTTGCCAT CCTATGGAAC TGCCTCGGTG AGTTTTCTCC 2640
TTCATTACAG AAACGGCTTT TTCAAAAATA TGGTATTGAT AATCCTGATA TGAATAAATT 2700
GCAGTTTCAT TTGATGCTCG ATGAGTTTTT CTAATCAGAA TTGGTTAATT GGTTGTAACA 2760

CTGGCAGAGC ATTACGCTGA CTTGACGGGA CGGCGGCTTT GTTGAATAAA TCGAACTTTT 2820
GCTGAGTTGA AGGATCAGAT CACGCATCTT CCCGACAACG CAGACCGTTC CGTGGCAAAG 2880
CAAAAGTTCA AAATCACCAA CTGGTCCACC TACAACAAAG CTCTCATCAA CCGTGGCTCC 2940
CTCACTTTCT GGCTGGATGA TGGGGCGATT CAGGCCTGGT ATGAGTCAGC AACACCTTCT 3000
TCACGAGGCA GACCTCAGCG CTCAAAGATG CAGGGGTAAA AGCTAACCGC ATCTTTACCG 3060
ACAAGGCATC CGGCAGTTCA ACAGATCGGG AAGGGCTGGA TTTGCTGAGG ATGAAGGTGG 3120
AGGAAGGTGA TGTCATTCTG GTGAAGAAGC TCGACCGTCT TGGCCGCGAC ACCGCCGACA 3180
TGATCCAACT GATAAAAGAG TTTGATGCTC AGGGTGTAGC GGTTCGGTTT ATTGACGACG 3240
GGATCAGTAC CGACGGTGAT ATGGGGCAAA TGGTGGTCAC CATCCTGTCG GCTGTGGCAC 3300
AGGCTGAACG CCGGAGGATC CAGTTCGATG TAACCCACTC GTGCACCCAA CTGATCTTCA 3360
GCATCTTTTA CTTTCACCAG CGTTTCTGGG TGAGCAAAAA CAGGAAGGCA AAATGCCGCA 3420
AAAAAGGGAA TAAGGGCGAC ACGGAAATGT TGAATACTCA TACTCTTCCT TTTTCAATAT 3480
TATTGAAGCA TTTATCAGGG TTATTGTCTC ATGAGCGGAT ACATATTTGA ATGTATTTAG 3540
AAAAATAAAC AAATAGGGGT TCCGCGCACA TTTCCCCGAA AAGTGCCACC TGACGTCTAA 3600
GAAACCATTA TTATCATGAC ATTAACCTAT AAAAATAGGC GTATCACGAG GCCCTTTCGT 3660
CTTCAAGTAT CTTTCCCTTT ATTTTTGCTG CGGTAAGTCG CATAAAAACC ATTCTTCATA 3720
ATTCAATCCA TTTACTATGT TATGTTCTGA GGGGAGTGAA AATTCCCCTA ATTCGATGAA 3780
GATTCTTGCT CAATTGTTAT CAGCTATGCG CCGACCAGAA CACCTTGCCG ATCAGCCAAA 3840
CGTCTCTTCA GGCCACTGAC TAGCGATAAC TTTCCCCACA ACGGAACAAC TCTCATTGCA 3900
TGGGATCATT GGGTACTGTG GGTTTAGTGG TTGTAAAAAC ACCTGACCGC TATCCCTGAT 3960
CAGTTTCTTG AAGGTAAACT CATCACCCCC AAGTCTGGCT ATGCAGAAAT CACCTGGCTC 4020
AACAGCCTGC TCAGGGTCAA CGAGAATTAA CATTCCGTCA GGAAAGCTTG GCTTGGAGCC 4080
TGTTGGTGCG GTCATGGAAT TACCTTCAAC CTCAAGCCAG AATGCAGAAT CACTGGCTTT 4140
TTTGGTTGTG CTTACCCATC TCTCCGCATC ACCTTTGGTA AAGGTTCTAA GCTTAGGTGA 4200
GAACATCCCT GCCTGAACAT GAGAAAAAAC AGGGTACTCA TACTCACTTC TAAGTGACGG 4260
CTGCATACTA ACCGCTTCAT ACATCTCGTA GATTTCTCTG GCGATTGAAG GGCTAAATTC 4320
TTCAACGCTA ACTTTGAGAA TTTTTGTAAG CAATGCGGCG TTATAAGCAT TTAATGCATT 4380
GATGCCATTA AATAAAGCAC CAACGCCTGA CTGCCCCATC CCCATCTTGT CTGCGACAGA 4440
TTCCTGGGAT AAGCCAAGTT CATTTTTCTT TTTTTCATAA ATTGCTTTAA GGCGACGTGC 4500
GTCCTCAAGC TGCTCTTGTG TTAATGGTTT CTTTTTTGTG CTCATACGTT AAATCTATCA 4560
CCGCAAGGGA TAAATATCTA ACACCGTGCG TGTTGACTAT TTTACCTCTG GCGGTGATAA 4620
TGGTTGCATG TACTAAGGAG GTTGTATGGA ACAACGCATA ACCCTGAAAG ATTATGCAAT 4680
GCGCTTTGGG CAAACCAAGA CAGCTAAAGA TCCTctagCT AGAATTCAGG CTTCTGCCGT 4740
TTTGGATTTA ACCGAAGATG ATTTCGATTT TCTGACGAGT AACAAAGTTT GGATTGCTAC 4800
TGACCGCTCT CGTGCTCGTC GCTGCGTTGA GGCTTGCGTT TATGGTACGC TGGACTTTGT 4860
GGGATACCCT CGCTTTCCTG CTCCTGTTGA GTTTATTGCT GCCGTCATTG CTTATTATGT 4920
TCATCCCGTC AACATTCAAA CGGCCTGTCT CATCATGGAA GGCGCTGAAT TTACGGAAAA 4980
CATTATTAAT GGCGTCGAGC GTCCGGTTAA AGCCGCTGAA TTGTTCGCGT TTACCTTGCG 5040
TGTACGCGCA GGAAACACTG ACGTTCTTAC TGACGCAGAA GAAAACGTGC GTCAAAAATT 5100
ACGTGCGGAA GGAGTGATGT AATGTCTAAA GGTAAAAAAC GTTCTGGCGC TCGCCCTGGT 5160
CGTCCGCAGC CGTTGCGAGG TACTAAAGGC AAGCGTAAAG GCGCTCGTCT TTGGTATGTA 5220
GGTGGTCAAC AATTTTAATT GCAGGGGCTT CGGCCCCTTA CTTGAGGATA AATTATGTCT 5280
AATATTCAAA CTGGCGCCGA GCGTATGCCG CATGACCTTT CCCATCTTGG CTTCCTTGCT 5340
GGTCAGATTG GTCGTCTTAT TACCATTTCA ACTACTCCGG TTATCGCTGG CGACTCCTTC 5400
GAGATGGACG CCGTTGGCGC TCTCCGTCTT TCTCCATTGC GTCGTGGCCT TGCTATTGAC 5460
TCTACTGTAG ACATTTTTAC TTTTTATGTC CCTCATCGTC ACGTTTATGG TGAACAGTGG 5520
ATTAAGTTCA TGAAGGATGG TGTTAATGCC ACTCCTCTCC CGACTGTTAA CACTACTGGT 5580
TATATTGACC ATGCCGCTTT TCTTGGCACG ATTAACCCTG ATACCAATAA AATCCCTAAG 5640
CATTTGTTTC AGGGTTATTT GAATATCTAT AACAACTATT TTAAAGCGCC GTGGATGCCT 5700
GACCGTACCG AGGCTAACCC TAATGAGAAT TCTCATGTTT GACAGCTTAT CATCGATAAG 5760
CTTTAATGCG GTAGTTTATC ACAGTTAAAT TGCTAACGCA GTCAGGCACC GTGTATGAAA 5820
TCTAACAATG CGCTCATCGT CATCCTCGGC ACCGTCACCC TGGATGCTGT AGGCATAGGC 5880
TTGGTTATGC CGGTACTGCC GGGCCTCTTG CGGGATATCG TCCATTCCGA CAGCATCGCC 5940
AGTCACTATG GCGTGCTGCT AGCGCTATAT GCGTTGATGC AATTTCTATG CGCACCCGTT 6000
CTCGGAGCAC TGTCCGACCG CTTTGGCCGC CGCCCAGTCC TGCTCGCTTC GCTACTTGGA 6060
GCCACTATCG ACTACGCGAT CATGGCGACC ACACCCGTCC TGTGGATCCG GATCAGCAGG 6120
TGGAAGAGGG ACTGGATTCC AAAGTTCTCA ATGCTGCTTG CTGTTCTTGA ATGGGGGGTC 6180

GTTGACGACG ACATGGCTCG ATTGGCGCGA CAAGTTGCTG CGATTCTCAC CAATAAAAAA 6240
CGCCCGGCGG CAACCGAGCG TTCTGAACAA ATCCAGATGG AGTTCTGAGG TCATTACTGG 6300
ATCGCCGGAT CTGAATTGCT ATGTTTAGTG AGTTGTATCT ATTTATTTTT CAATAAATAC 6360
AATTGGTTAT GTGTTTTGGG GGCGATCGTG AGGCAAAGAA AACCCGGCGC TGAGGCCGGA 6420
AGCATAAAGT GTAAAGCCTG GGGTGCCTAA TGAGTGAGCT AACTCACATT AATTGCGTTG 6480
CGCTCACTGC CCGCTTTCCA GTCGGGAAAC CTGTCGTGCC AGCTGCATTA ATGAATCGGC 6540
CAACGCGCGG GGAGAGGCGG TTTGCGTATT GGGCGCCAGG GTGGTTTTTC TTTTCACCAG 6600
TGAGACGGGC AACAGCTGAT TGCCCTTCAC CGCCTGGCCC TGAGAGAGTT GCAGCAAGCG 6660
GTCCACGCTG GTTTGCCCCA GCAGGCGAAA ATCCTGTTTG ATGGTGGTTG ACGGCGGGAT 6720
ATAACATGAG CTGTCTTCGG TATCGTCGTA TCCCACTACC GAGATATCCG CACCAACGCG 6780
CAGCCCGGAC TCGGTAATGG CGCGCATTGC GCCCAGCGCC ATCTGATCGT TGGCAACCAG 6840
CATCGCAGTG GGAACGATGC CCTCATTCAG CATTTGCATG GTTTGTTGAA AACCGGACAT 6900
GGCACTCCAG TCGCCTTCCC GTTCCGCTAT CGGCTGAATT TGATTGCGAG TGAGATATTT 6960
ATGCCAGCCA GCCAGACGCA GACGCGCCGA GACAGAACTT AATGGGCCCG CTAACAGCGC 7020
GATTTGCTGG TGACCCAATG CGACCAGATG CTCCACGCCC AGTCGCGTAC CGTCTTCATG 7080
GGAGAAAATA ATACTGTTGA TGGGTGTCTG GTCAGAGACA TCAAGAAATA ACGCCGGAAC 7140
ATTAGTGCAG GCAGCTTCCA CAGCAATGGC ATCCTGGTCA TCCAGCGGAT AGTTAATGAT 7200
CAGCCCACTG ACGCGTTGCG CGAGAAGATT GTGCACCGCC GCTTTACAGG CTTCGACGCC 7260
GCTTCGTTCT ACCATCGACA CCACCACGCT GGCACCCAGT TGATCGGCGC GAGATTTAAT 7320
CGCCGCGACA ATTTGCGACG GCGCGTGCAG GGCCAGACTG GAGGTGGCAA CGCCAATCAG 7380
CAACGACTGT TTGCCCGCCA GTTGTTGTGC CACGCGGTTG GGAATGTAAT TCAGCTCCGC 7440
CATCGCCGCT TCCACTTTTT CCCGCGTTTT CGCAGAAACG TGGCTGGCCT GGTTCACCAC 7500
GCGGGAAACG GTCTGATAAG AGACACCGGC ATACTCTGCG ACATCGTATA ACGTTACTGG 7560
TTTCACATTC ACCACCCTGA ATTGACTCTC TTCCGGCGCT ATCATGCCAT ACCGCGAAAG 7620
GTTTTGCGCC ATTCGATGGT G 7641

SEQ IDNO:6
ART DER SEQUENZ:Nucleotidsequenz (pKSEL5)
SEQUENZLÄNGE:3681 Basenpaare

STRANGFORM:Einzelstrang

AAATTGTAAA CGTTAATATT TTGTTAAAAT TCGCGTTAAA TTTTTGTTAA ATCAGCTCAT 60
TTTTTAACCA ATAGGCCGAA ATCGGCAAAA TCCCTTATAA ATCAAAAGAA TAGACCGAGA 120
TAGGGTTGAG TGTTGTTCCA GTTTGGAACA AGAGTCCACT ATTAAAGAAC GTGGACTCCA 180
ACGTCAAAGG GCGAAAAACC GTCTATCAGG GCGATGGCCC ACTACGTGAA CCATCACCCT 240
AATCAAGTTT TTTGGGGTCG AGGTGCCGTA AAGCACTAAA TCGGAACCCT AAAGGGAGCC 300
CCCGATTTAG AGCTTGACGG GGAAAGCCGG CGAACGTGGC GAGAAAGGAA GGGAAGAAAG 360
CGAAAGGAGC GGGCGCTAGG GCGCTGGCAA GTGTAGCGGT CACGCTGCGC GTAACCACCA 420
CACCCGCCGC GCTTAATGCG CCGCTACAGG GCGCGTCCCA TTCGCCATTC AGGCTACGCA 480
ACTGTTGGGA AGGGCGATCG GTGCGGGCCT CTTCGCTATT ACGCCAGCTG GCGAAGGGGG 540
GATGTGCTGC AAGGCGATTA AGTTGGGTAA CGCCAGGGTT TTCCCAGTCA CGACGTTGTA 600
AAACGACGGC CAGTGAATTG TAATACGACT CACTATAGGG CGAATTGGAG CTCCACCGCG 660
GTGGCGGCCG CTCTAGTATG GTGCACTCTC AGTACAATCT GCTCTGATGC CGCATAGTTA 720
AGCCAGTATA TACACTCCGC TATCGCTACG TGACTGGGTC ATGGCTGCGC CCCGACACCC 780
GCCAACACCC GCTGACGCGC CCTGACGGGC TTGTCTGCTC CCGGCATCCG CTTACAGACA 840
AGCTGTGACC GTCTCCGGGA GCTGCATGTG TCAGAGGTTT TCACCGTCAT CACCGAAACG 900
CGCGAGGCAG TAAGGTCGGA TGCTTTGTGA GCAATTCGTC CCTTAAGTAA GCAATTGCTG 960
TAAAGTCGTC ACTGTGCGGA TCACCGCTTC CAGTAGCGAC AGAAGCAATT GATTGGTAAA 1020
TTTCGAGAGA AAGATCGCGA GGAAGATCAA TACATAAAGA GTTGAACTTC TTTGTTGTCT 1080
TCGACATGGG TAATCCTCAT GTTTGAATGG CCCTAGAGGA TCCGGCCAAG CTTGCATGCC 1140
TGCAGGTCGA CTCTAGAGGA TCCCCGACGC TCGACGCCAT TAATAATGTT TTCCGTAAAT 1200
TCAGCGCCTT CCATGATGAG ACAGGCCGTT TGAATGTTGA CGGGATGAAC ATAATAAGCA 1260
ATGACGGCAG CAATAAACTC AACAGGAGCA GGAAAGCGAG GGTATCCCAC AAAGTCCAGC 1320
GTACCATAAA CGCAAGCCTC AACGCAGCGA CGAGCACGAG AGCGGTCAGT AGCAATCCAA 1380
ACTTTGTTAC TCGTCAGAAA ATCGAAATCA TCTTCGGTTA AATCCAAAAC GGCAGAAGCC 1440
TGAATGAGAA TTCGACCTCG AGGGGGGGCC CGGTACCCAG CTTTTGTTCC CTTTAGTGAG 1500
GGTTAATTCC GAGCTTGGCG TAATCATGGT CATAGCTGTT TCCTGTGTGA AATTGTTATC 1560
CGCTCACAAT TCCACACAAC ATAGGAGCCG GAAGCATAAA GTGTAAAGCC TGGGGTGCCT 1620
AATGAGTGAG GTAACTCACA TTAATTGCGT TGCGCTCACT GCCCGCTTTC CAGTCGGGAA 1680
ACCTGTCGTG CCAGCTGCAT TAATGAATCG GCCAACGCGC GGGGAGAGGC GGTTTGCGTA 1740
TTGGGCGCTC TTCCGCTTCC TCGCTCACTG ACTCGCTGCG CTCGGTCGTT CGGCTGCGGC 1800
GAGCGGTATC AGCTCACTCA AAGGCGGTAA TACGGTTATC CACAGAATCA GGGGATAACG 1860
CAGGAAAGAA CATGTGAGCA AAAGGCCAGC AAAAGGCCAG GAACCGTAAA AAGGCCGCGT 1920
TGCTGGCGTT TTTCCATAGG CTCGGCCCCC CTGACGAGCA TCACAAAAAT CGACGCTCAA 1980
GTCAGAGGTG GCGAAACCCG ACAGGACTAT AAAGATACCA GGCGTTCCCC CCTGGAAGCT 2040
CCCTCGTGCG CTCTCCTGTT CCGACCCTGC CGCTTACCGG ATACCTGTCC GCCTTTCTCC 2100
CTTCGGGAAG CGTGGCGCTT TCTCAATGCT CACGCTGTAG GTATCTCAGT TCGGTGTAGG 2160
TCGTTCGCTC CAAGCTGGGC TGTGTGCACG AACCCCCCGT TCAGCCCGAC CGCTGCGCCT 2220
TATCCGGTAA CTATCGTCTT GAGTCCAACC CGGTAAGACA CGACTTATCG CCACTGGCAG 2280
CAGCCACTGG TAACAGGATT AGCAGAGCGA GGTATGTAGG CGGTGCTACA GAGTTCTTGA 2340
AGTGGTGGCC TAACTACGGC TACACTAGAA GGACAGTATT TGGTATCTGC GCTCTGCTGA 2400
AGCCAGTTAC CTTCGGAAAA AGAGTTGGTA GCTCTTGATC CGGCAAACAA ACCACCGCTG 2460
GTAGCGGTGG TTTTTTTGTT TGCAAGCAGC AGATTACGCG CAGAAAAAAA GGATCTCAAG 2520
AAGATCCTTT GATCTTTTCT ACGGGGTCTG ACGCTCAGTG GAACGAAAAC TCACGTTAAG 2580
GGATTTTGGT CATGAGATTA TCAAAAAGGA TCTTCACCTA GATCCTTTTA AATTAAAAAT 2640
GAAGTTTTAA ATCAATCTAA AGTATATATG AGTAAACTTG GTCTGACAGT TACCAATGCT 2700

TAATCAGTGA GGCACCTATC TCAGCGATCT GTCTATTTCG TTCATCCATA GTTGCCTGAC 2760
TGCCCGTCGT GTAGATAACT ACGATACGGG AGGGCTTACC ATCTGGCCCC AGTGCTGCAA 2820
TGATACCGCG AGACCCACGC TCACCGGCTC CAGATTTATC AGCAATAAAC CAGCCAGCCG 2880
GAAGGGCCGA GCGCAGAAGT GGTCCTGCAA CTTTATCCGC CTCCATCCAG TCTATTAATT 2940
GTTGCCGGGA AGCTAGAGTA AGTAGTTCGC CAGTTAATAG TTTGCGCAAC GTTGTTGCCA 3000
TTGCTACAGG CATCGTGGTG TCACGCTCGT CGTTTGGTAT GGCTTCATTC AGCTCCGGTT 3060
CCCAACGATC AAGGCGAGTT ACATGATCCC CCATGTTGTG AAAAAAGCGG TTAGCTCCTT 3120
TCGGTCCTCC GATCGTTGTC AGAAGTAAGT TGGCCGCAGT GTTATCACTC ATGCTTATGG 3180
CAGCACTGCA TAATTCTCTT ACTGTCATGC CATCCGTAAG ATGCTTTTCT GTGACTGGTG 3240
AGTACTCAAC CAAGTCATTC TGAGAATAGT GTATGCGGCG ACCGAGTTGC TCTTGCCCGG 3300
CGTCAATACG GGATAATACC GCGCCACATA GCAGAACTTT AAAAGTGCTC ATCATTGGAA 3360
AACGTTCTTC GGGGCGAAAA CTCTCAAGGA TCTTACCGCT GTTGAGATCC AGTTCGATGT 3420
AACCCACTCG TGCACCCAAC TGATCTTCAG CATCTTTTAC TTTCACCAGC GTTTCTGGGT 3480
GAGCAAAAAC AGGAAGGCAA AATGCCGCAA AAAAGGGAAT AAGGGCGACA CGGAAATGTT 3540
GAATACTCAT ACTCTTCCTT TTTCAATATT ATTGAAGCAT TTATCAGGGT TATTGTCTCA 3600
TGAGCGGATA CATATTTGAA TGTATTTAGA AAAATAAACA AATAGGGGTT CCGCGCACAT 3660
TTCCCCGAAA AGTGCCACCT G 3681

## Patentansprüche (Patentansprüche für folgende(n) Vertragsstaat(en) : AT, BE, CH, LI, DE, DK, FR, GB, GR, IT, LU, NL, SE)

1. An die Membran von gramnegativen Bakterien gebundenes rekombinantes Protein, erhältlich durch Expression einer rekombinanten DNA, in diesen gramnegativen Bakterien, welche eine erste DNA-Sequenz (DNA-Targetsequenz), welche für mindestens eine hydrophobe, nicht lytisch wirkende membranintegrierende Proteindomäne, die eine alphahelicale Struktur besitzt und aus 14 bis 20 Aminosäuren besteht, die N- und C-terminal vonje 2 bis 30 beliebigen Aminosäuren flankiert sein können, codiert, eine zweite DNA-Sequenz (DNA-Proteinsequenz), die für ein rekombinantes Protein codiert, sowie eine dritte unter davon getrennter Kontrolle stehenden DNA-Sequenz (DNA-Lysegen), die für ein lytisch wirkendes Membranprotein aus Bacteriophagen oder ein lytisch wirkendes Toxin-release-Gen oder für deren lytisch wirkenden Teile codiert, enthält und Gewinnung des trägergebundenen rekombinanten Proteins aus derKulturbrühe.

2. Protein nach Anspruch 1 dadurch gekennzeichnet, daß das Fusionsprotein mindestens eine weitere Proteindomäne enthält, die aus 10 bis 16 Aminosäuren besteht und eine β-Faltblatt-Sekundärstruktur besitzt.

3. Protein nach den Ansprüchen 1 oder2 dadurch gekennzeichnet, daß die Proteindomäne, dieAminosäuren 1 bis 54 aus dem Protein E des Phagen PhiX174, die Aminosäuren 21 bis 75 aus dem Protein L des Phagen MS2 und/oder eine Aminsoäuresequenz, die aus diesen Sequenzen durch Aminosäureaustausch erhältlich ist und eine analoge Protein-Sekundärstruktur besitzt, enthält.

4. Protein nach den Ansprüchen 1 bis 3 dadurch gekennzeichnet, daß die Proteindomänen und das rekombinante Protein durch eine hydrophile Aminosäuresequenz mit 2 bis 100 Aminosäuren und ungeordneter Sekundärstruktur verbunden sind.

5. Protein nach den Ansprüchen 1 bis 4 dadurch gekennzeichnet, daß das rekombinante Protein eine antigene Struktur aufweist.

6. Protein nach den Ansprüchen 1 bis 5 dadurch gekennzeichnet, daß an das rekombinante Protein ein nicht kovalent bindender Bindungspartner für dieses Protein, an dem gegebenenfalls noch weitere Substanzen kovalent oder nicht kovalent gebunden sind, gebunden ist.

7. Protein nach Anspruch 6 dadurch gekennzeichnet, daß das rekombinante Protein Streptavidin oderAvidin ist.

8. Protein nach den Ansprüchen 6 und 7 dadurch gekennzeichnet, daß der nicht kovalente Bindungspartner ein biotinyliertes Antigen ist.

9. Rekombinante DNA, die eine erste DNA-Sequenz (DNA-Targetsequenz), welche für mindestens eine hydrophobe, nicht lytisch wirkende membranintegrierende Proteindomäne, die eine alphahelicale Struktur besitzt und aus 14 bis 20 Aminosäuren besteht, die N-und C-terminal von je 2 - 30 beliebigen Aminosäuren flankiert sein können, codiert, eine zweite DNA-Sequenz (DNA-Proteinsequenz), die für ein rekombinantes Protein kodiert, sowie eine dritte unter davon getrennter Kontrolle stehenden DNA-Sequenz (DNA-Lysegen), die für ein lytisch wirkendes Membranprotein aus Bacteriophagen oder ein lytisch wirkendes Toxin-release-Gen oder für deren lytisch wirkenden Teile kodiert, enthält.

10. Verfahren zur Herstellung eines an die Membran von gramnegativen Bakterien gebundenen rekombinanten Proteins,
dadurch gekennzeichnet,
daß in diesen gramnegativen Bakterien eine rekombinante DNA, die eine erste DNA-Sequenz (DNA-Targetsequenz), welche für mindestens eine hydrophobe, nicht lytisch wirkende membranintegrierende Proteindomäne, die eine alphahelicale Struktur besitzt und aus 14 bis 20 Aminosäuren besteht, die N- und C-terminal von je 2 bis 30 beliebigen Aminosäuren flankiert sein können, codiert, eine zweite DNA-Sequenz (DNA-Proteinsequenz), die für ein rekombinantes Protein codiert, sowie eine dritte unter davon getrennter Kontrolle stehenden DNA-Sequenz (DNA- Lysegen), die für ein lytisch wirkendes Membranprotein aus Bacteriophagen oder ein lytisch wirkendes Toxin-release-Gen oder für deren lytisch wirkenden Teile codiert, enthält exprimiert werden und das trägergebundene rekombinante Protein aus der Kulturbrühe gewonnen wird.

11. Verfahren nach Anspruch 10, dadurch gekennzeichnet, daß mindestens ein weiteres Gen eines rekombinanten Proteins exprimiert wird.

12. Verfahren nach den Ansprüchen 10 oder 11 dadurch gekennzeichnet, daß bei der Kultivierung die Aktivität des lytischen Membranproteins inhibiertoderdie Expression des lytisch wirkenden Membranproteins oder Toxingens reprimiert wird und zu einem gewünschten Zeitpunkt die Inhibierung oder Repression aufgehoben wird.

13. Verfahren nach den Ansprüchen 10 - 12 dadurch gekennzeichnet, daß das Protein mit einem gegebenenfalls derivatisierten Bindungspartner für das Protein inkubiert und das entstandene an die Membran von gramnegativen Bakterien gebundene Konjugat isoliert wird.

14. Verfahren zur Herstellung von Antikörpern dadurch gekennzeichnet, daß ein Säugetier mit einem Protein nach den Ansprüchen 1 bis 8 immunisiertwird und die Antikörper aus dem Serum oder der Milz gewonnen werden.

15. Verfahren zur Herstellung von Antikörpern nach Anspruch 14 dadurch gekennzeichnet, daß B-Lymphozyten der immunisierten Tiere in Gegenwart von transfomierenden Agenzien, mit einer geeigneten Zellinie fusioniert werden, die gewünschte Antikörper produzierende Zellinie kloniert und kultiviert wird und aus den Zellen oder dem Kulturüberstand die Antikörper gewonnen werden.

16. Verwendung der trägergebundenen Proteine nach den Ansprüchen 1 - 8 zur Herstellung von Vakzinen.

## Patentansprüche (Patentansprüche für folgende(n) Vertragsstaat(en) : ES)

1. Verfahren zur Herstellung eines an die Membran von gramnegativen Bakterien gebundenen rekombinanten Proteins,
dadurch gekennzeichnet,
daß in diesen gramnegativen Bakterien eine rekombinante DNA, die eine erste DNA-Sequenz (DNA-Targetsequenz), welche für mindestens eine hydrophobe, nicht lytisch wirkende membranintegrierende Proteindomäne, die eine aliphahelicale Struktur besitzt und aus 14 bis 20 Aminosäuren besteht, die N- und C-terminal von je 2 bis 30 beliebigen Aminosäuren flankiert sein können, codiert, eine zweite DNA-Sequenz (DNA-Proteinsequenz), die für ein rekombinantes Protein codiert, sowie eine dritte unter davon getrennter Kontrolle stehenden DNA-Sequenz (DNA-Lysegen), die fürein lytisch wirkendes Membranprotein aus Bacteriophagen oder ein lytisch wirkendes Toxin-release-Gen oder für deren lytisch wirkenden Teile codiert, enthält exprimiert werden und das trägergebundene rekombinante Protein aus der Kulturbrühe gewonnen wird.

2. Verfahren nach Anspruch 1,
dadurch gekennzeichnet,
daß mindestens ein weiteres Gen eines rekombinanten Proteins exprimiert wird.

3. Verfahren nach den Ansprüchen 1 oder 2,
dadurch gekennzeichnet,
daß bei der Kultivierung die Aktivität des lytischen Membranproteins inhibiert oder die Expression des lytisch wirkenden Membranproteins oder Toxingens reprimiert wird und zu einem gewünschten Zeitpunkt die Inhibierung oder Repression aufgehoben wird.

4. Verfahren nach den Ansprüchen 1 bis 3,
dadurch gekennzeichnet,
daß das Protein mit einem gegebenenfalls derivatisierten Bindungspartner für das Protein inkubiert und das entstandene an die Membran von gramnegativen Bakterien gebundene Konjugat isoliert wird.

5. Verfahren nach Anspruch 1,
dadurch gekennzeichnet,
daß das Fusionsprotein mindestens eine weitere Proteindomäne enthält, die aus 10 bis 16 Aminosäuren besteht und eine β-Faltblatt-Sekundärstruktur besitzt.

6. Verfahren nach Anspruch 1,
dadurch gekennzeichnet,
daß man für die Proteindomäne die Aminosäuren 1 bis 54 aus dem Protein E des Phagen PhiX174 oder die Aminosäuren 21 bis 75 aus dem Protein L des Phagen MS2 und/oder eine Aminosäuresequenz, die aus diesen Sequenzen durch Aminosäureaustausch erhältlich ist und eine analoge Protein-Sekundärstruktur besitzt, verwendet.

7. Verfahren nach Anspruch 1,
dadurch gekennzeichnet,
daß die Proteindomänen und das rekombinante Protein durch eine hydrophile Aminosäuresequenz mit 2 bis 100 Aminosäuren und ungeordneter Sekundärstruktur verbunden werden.

8. Verfahren nach Anspruch 1,
dadurch gekennzeichnet,
daß das rekombinante Protein eine antigene Struktur aufweist.

9. Verfahren nach Anspruch 1,
dadurch gekennzeichnet,
daß man an das rekombinante Protein einen nicht kovalent bindenden Bindungspartnerfür dieses Protein, an dem gegebenenfalls noch weitere Substanzen kovalent oder nicht kovalent gebunden sind, bindet.

10. Verfahren nach Anspruch 9,
dadurch gekennzeichnet,
daß man als rekombinantes Protein Streptavidin oder Avidin verwendet.

11. Protein nach den Ansprüchen 9 und 10,
dadurch gekennzeichnet,
daß der nicht kovalente Bindungspartner ein biotinyliertes Antigen ist.

12. Verfahren zur Herstellung von Antikörpern,
dadurch gekennzeichnet,
daß ein Säugetier mit einem Protein, das nach den Ansprüchen 1 bis 11 erhalten wurde, immunisiert wird und die Antikörper aus dem Serum oder der Milz gewonnen werden.

13. Verfahren zur Herstellung von Antikörpern nach Anspruch 12,
dadurch gekennzeichnet,
daß B-Lymphozyten der immunisierten Tiere in Gegenwart von transfomierenden Agenzien, mit einer geeigneten Zellinie fusioniert werden, die gewünschte Antikörper produzierende Zellinie kloniert und kultiviert wird und aus den Zellen oder dem Kulturüberstand die Antikörper gewonnen werden.

14. Verwendung der trägergebundenen Proteine die nach den Ansprüche 1 bis 11 erhalten werden zur Herstellung von Vakzinen.

## Claims (Claims for the following Contracting State(s) : AT, BE, CH, Ll, DE, DK, FR, GB, GR, IT, LU, NL, SE)

1. Recombinant protein bound to the membrane of gram-negative bacteria obtainable by expression of a recombinant DNA in these gram-negative bacteria which contains a first DNA sequence (DNA target sequence) that codes for at least one hydrophobic, non-lytically active, membrane-integrating protein domain which has an alpha-helical structure and is composed of 14 to 20 amino acids which can be flanked N- and C-terminally by 2 to 30 arbitrary amino acids in each case, a second DNA sequence (DNA protein sequence) that codes for a recombinant protein as well as a third DNA sequence (DNA lysis gene) under separate control from this that codes for a lytically-active membrane protein from bacteriophages or a lytically-active toxin release gene or for their lytically active parts and isolation of the carrier-bound recombinant protein from the culture broth.

2. Protein as claimed in claim 1, wherein the fusion protein contains at least one further protein domain which consists of 10 to 16 amino acids and has a ß-pleated sheet secondary structure.

3. Protein as claimed in claims 1 or 2, wherein the protein domain contains the amino acids 1 to 54 from the protein E of the phage PhiX174, the amino acids 21 to 75 from the protein L of the phage MS2 and/or an amino acid sequence which is obtainable from these sequences by amino acid substitution and has an analogous protein secondary structure.

4. Protein as claimed in claims 1 to 3, wherein the protein domains and the recombinant protein are linked by a hydrophilic amino acid sequence with 2 to 100 amino acids and a disordered secondary structure.

5. Protein as claimed in claims 1 to 4, wherein the recombinant protein has an antigenic structure.

6. Protein as claimed in claims 1 to 5, wherein a non-covalently binding binding partner for this protein to which, if desired, further substances are bound covalently or non-covalently, is bound to the recombinant protein

7. Protein as claimed in claim 6, wherein the recombinant protein is streptavidin or avidin.

8. Protein as claimed in claims 6 and 7, wherein the non-covalent binding partner is a biotinylated antigen.

9. Recombinant DNA which contains a first DNA sequence (DNA target sequence), which codes for at least one hydrophobic, non-lytically active, membrane-integrating protein domain which has an alpha-helical structure and is composed of 14 to 20 amino acids which can be flanked N- and C-terminally by 2 to 30 arbitrary amino acids in each case, a second DNA sequence (DNA protein sequence) that codes for a recombinant protein as well as a third DNA sequence (DNA lysis gene) under separate control from this that codes for a lytically-active membrane protein from bacteriophages or a lytically-active toxin release gene or for their lytically active parts.

10. Process for the production of a recombinant protein bound to the membrane of gram-negative bacteria, wherein a recombinant DNA which contains a first DNA sequence (DNA target sequence) that codes for at least one hydrophobic, non-lytically active, membrane-integrating protein domain which has an alpha-helical structure and is composed of 14 to 20 amino acids which can be flanked N- and C-terminally by 2 to 30 arbitrary amino acids in each case, a second DNAsequence (DNA protein sequence) that codes for a recombinant protein as well as a third DNA sequence (DNA lysis gene) under separate control from this that codes for a lytically-active membrane protein from bacteriophages or a lytically-active toxin release gene or for their lytically active parts is expressed in these gram-negative bacteria and the carrier-bound recombinant protein is isolated from the culture broth.

11. Process as claimed in claim 10, wherein at least one further gene of a recombinant protein is expressed.

12. Process as claimed in claims 10 or 11, wherein during the culture the activity of the lytic membrane protein is inhibited or the expression of the lytically active membrane protein or toxin gene is repressed and the inhibition or repression is abolished at a desired time.

13. Process as claimed in claims 10-12, wherein the protein is incubated with a binding partnerforthe protein which is derivatized if desired and the conjugate bound to the membrane of gram-negative bacteria which is formed is isolated.

14. Process for the production of antibodies, wherein a mammal is immunized with a protein as claimed in claims 1 to 8 and the antibodies are isolated from the serum or the spleen.

15. Process for the production of antibodies as claimed in claim 14, wherein the B lymphocytes of the immunized animals are fused with a suitable cell line in the presence of transforming agents, the cell line producing the desired antibodies is cloned and cultured, and the antibodies are isolated from the cells or the culture supernatant.

16. Use of the carrier-bound proteins as claimed in claims 1 - 8 for the production of vaccines.

## Claims (Claims for the following Contracting State(s) : ES)

1. Process for the production of a recombinant protein bound to the membrane of gram-negative bacteria,
wherein
a recombinant DNA which contains a first DNA sequence (DNA target sequence) that codes for at least one hydrophobic, non-lytically active, membrane-integrating protein domain which has an alpha-helical structure and is composed of 14 to 20 amino acids which can be flanked N- and C-terminally by 2 to 30 arbitrary amino acids in each case, a second DNA sequence (DNA protein sequence) that codes for a recombinant protein as well as a third DNA sequence (DNA lysis gene) under separate control from this that codes for a lytically-active membrane protein from bacteriophages or a lytically-active toxin release gene or for their lytically active parts is expressed in these gram-negative bacteria and the carrier-bound recombinant protein is isolated from the culture broth.

2. Process as claimed in claim 1,
wherein
at least one further gene of a recombinant protein is expressed.

3. Process as claimed in claims 1 or 2,
wherein
during the culture the activity of the lytic membrane protein is inhibited or the expression of the lytically active membrane protein or toxin gene is repressed and the inhibition or repression is abolished at a desired time.

4. Process as claimed in claims 1 to 3,
wherein
the protein is incubated with a binding partner for the protein which is derivatized if desired and the conjugate bound to the membrane of gram-negative bacteria which is formed is isolated.

5. Process as claimed in claim 1,
wherein
the fusion protein contains at least one further protein domain which consists of 10 to 16 amino acids and has a ß-pleated sheet secondary structure.

6. Process as claimed in claim 1,
wherein
for the protein domain one uses the amino acids 1 to 54 from the protein E of the phage PhiX174 or the amino acids 21 to 75 from the protein L of the phage MS2 and/or an amino acid sequence which is obtainable from these sequences by amino acid substitution and has an analogous protein secondary structure.

7. Process as claimed in claim 1,
wherein
the protein domains and the recombinant protein are linked by a hydrophilic amino acid sequence with 2 to 100 amino acids and a disordered secondary structure.

8. Process as claimed in claim 1,
wherein
the recombinant protein has an antigenic structure.

9. Process as claimed in claim 1,
wherein
a non-covalently binding binding partner for this protein to which, if desired, further substances are bound covalently or non-covalently, is bound to the recombinant protein

10. Process as claimed in claim 9,
wherein
streptavidin or avidin is used as the recombinant protein.

11. Protein as claimed in claims 9 and 10,
wherein
the non-covalent binding partner is a biotinylated antigen.

12. Process for the production of antibodies,
wherein
a mammal is immunized with a protein which was obtained according to claims 1 to 11 and the antibodies are isolated from the serum or the spleen.

13. Process for the production of antibodies as claimed in claim 12,
wherein
the B lymphocytes of the immunized animals are fused with a suitable cell line in the presence of transforming agents, the cell line producing the desired antibodies is cloned and cultured, and the antibodies are isolated from the cells or the culture supernatant.

14. Use of the carrier-bound proteins which are obtained according to claims 1 - 11 for the production of vaccines.

## Revendications (Revendications pour l'(les) Etat(s) contractant(s) suivant(s) : suivants : AT, BE, CH, LI, DE, DK, FR, GB, GR, IT, LU, NL, SE)

1. Protéine recombinée liée à la membrane de bactéries gram-négatives, pouvant être obtenue par l'expression, dans ces bactéries gram-négatives, d'un ADN recombiné qui contient une première séquence d'ADN (ADN correspondant à la séquence cible) qui code pour au moins un domaine protéinique hydrophobe s'intégrant dans la membrane sans effet lytique, possédant une structure d'hélice a et constitué de 14 à 20 aminoacides qui peuvent être flanqués en position N- et C-terminale par, à chaque fois, 2 à 30 aminoacides quelconques, une deuxième séquence d'ADN (ADN correspondant à la séquence de la protéine) qui code pour une protéine recombinée, ainsi qu'une troisième séquence d'ADN (ADN correspondant à un gène de lyse), située sous un contrôle séparé, qui code pour une protéine membranaire à activité lytique de bactériophages ou un gène de libération d'une toxine à activité lytique ou leurs parties à activité lytique, et la récupération de la protéine recombinée liée à un support à partir du bouillon de culture.

2. Protéine selon la revendication 1, caractérisée en ce que la protéine de fusion contient au moins un autre domaine protéinique qui est constitué de 10 à 16 aminoacides et qui possède une structure secondaire repliée en ß.

3. Protéine selon les revendications 1 ou 2, caractérisée en ce que le domaine protéinique contient les aminoacides 1 à 54 de la protéine E du phage PhiX174, les aminoacides 21 à 75 de la protéine L du phage MS2 et/ou une séquence d'aminoacides qui peut être obtenue à partir de ces séquences par échange d'aminoacides et qui possède une structure secondaire protéinique analogue.

4. Protéine selon les revendications 1 à 3, caractérisée en ce que les domaines protéiniques et la protéine recombinée sont liés par l'intermédiaire d'une séquence d'aminoacides hydrophile ayant 2 à 100 aminoacides et une structure secondaire désordonnée.

5. Protéine selon les revendications 1 à 4, caractérisée en ce que la protéine recombinée présente une structure antigénique.

6. Protéine selon les revendications 1 à 5, caractérisée en ce que la protéine recombinée est liée à un partenaire de liaison ne formant pas de liaison covalente avec cette protéine, auquel sont éventuellement liées d'autres substances de façon covalente ou non covalente.

7. Protéine selon la revendication 6, caractérisée en ce que la protéine recombinée est la streptavidine ou l'avidine.

8. Protéine selon les revendications 6 et 7, caractérisée en ce que le partenaire de liaison non covalente est un antigène biotinylé.

9. ADN recombiné qui contient une première séquence d'ADN (ADN correspondant à la séquence cible) qui code pour au moins un domaine protéinique hydrophobe s'intégrant dans la membrane sans effet lytique, possédant une structure d'hélice a et constitué de 14 à 20 aminoacides qui peuvent être flanqués en position N- et C-terminale par, à chaque fois, 2 à 30 aminoacides quelconques, une deuxième séquence d'ADN (ADN correspondant à la séquence de la protéine) qui code pour une protéine recombinée, ainsi qu'une troisième séquence d'ADN (ADN correspondant à un gène de lyse), située sous un contrôle séparé, qui code pour une protéine membranaire de bactériophages à activité lytique ou un gène de libération d'une toxine à activité lytique ou leurs parties à activité lytique.

10. Procédé de préparation d'une protéine recombinée liée à la membrane de bactéries gram-négatives, caractérisé en ce que l'on faits'exprimer, dans ces bactéries gram-négatives, un ADN recombiné qui contient une première séquence d'ADN (ADN correspondant à la séquence cible) qui code pour au moins un domaine protéinique hydrophobe s'intégrant dans la membrane sans effet lytique, possédant une structure d'hélice a et constitué de 14 à 20 aminoacides qui peuvent être flanqués en position N- et C-terminale par, à chaque fois, 2 à 30 aminoacides quelconques, une deuxième séquence d'ADN (ADN correspondant à la séquence de la protéine) qui code pour une protéine recombinée, ainsi qu'une troisième séquence d'ADN (ADN correspondant à un gène de lyse), située sous un contrôle séparé, qui code pour une protéine membranaire de bactériophages à activité lytique ou un gène de libération d'une toxine à activité lytique ou leurs parties à activité lytique, et on récupère la protéine recombinée liée à un support à partir du bouillon de culture.

11. Procédé selon la revendication 10, caractérisé en ce que l'on fait s'exprimer au moins un autre gène d'une protéine recombinée.

12. Procédé selon les revendications 10 ou 11, caractérisé en ce que, lors de la culture, on inhibe l'activité de la protéine membranaire lytique ou on réprime l'expression de la protéine membranaire ou du gène de toxine à activité lytique, et, à un moment désiré, on supprime l'inhibition ou la répression.

13. Procédé selon les revendications 10 à 12, caractérisé en ce que l'on fait incuber la protéine avec un partenaire de liaison pour la protéine, éventuellement mis sous forme d'un dérivé, et on isole le conjugué formé lié à la membrane de bactéries gram-négatives.

14. Procédé de préparation d'anticorps, caractérisé en ce que l'on immunise un mammifère avec une protéine selon les revendications 1 à 8, et on récupère les anticorps à partir du sérum ou de la rate.

15. Procédé de préparation d'anticorps selon la revendication 14, caractérisé en ce que l'on fusionne des lymphocytes B des animaux immunisés avec une lignée cellulaire convenable en présence d'agents de transformation, on clone et on cultive la lignée cellulaire productrice d'anticorps désirée et on récupère les anticorps à partir des cellules ou du surnageant de culture.

16. Utilisation des protéines liées à un support selon les revendications 1 à 8 pour la préparation de vaccins.

## Revendications (Revendications pour l'(les) Etat(s) contractant(s) suivant(s) : suivant : ES)

1. Procédé de préparation d'une protéine recombinée liée à la membrane de bactéries gram-négatives, caractérisé en ce que l'on fait s'exprimer, dans ces bactéries gram-négatives, un ADN recombiné qui contient une première séquence d'ADN (ADN correspondant à la séquence cible) qui code pour au moins un domaine protéinique hydrophobe s'intégrant dans la membrane sans effet lytique, possédant une structure d'hélice a et constitué de 14 à 20 aminoacides qui peuvent être flanqués en position N- et C-terminale par, à chaque fois, 2 à 30 aminoacides quelconques, une deuxième séquence d'ADN (ADN correspondant à la séquence de la protéine) qui code pour une protéine recombinée, ainsi qu'une troisième séquence d'ADN (ADN correspondant à un gène de lyse), située sous un contrôle séparé, qui code pour une protéine membranaire de bactériophages à activité lytique ou un gène de libération d'une toxine à activité lytique ou leurs parties à activité lytique, et on récupère la protéine recombinée liée à un support à partir du bouillon de culture.

2. Procédé selon la revendication 1, caractérisé en ce que l'on fait s'exprimer au moins un autre gène d'une protéine recombinée.

3. Procédé selon les revendications 1 ou 2, caractérisé en ce que, lors de la culture, on inhibe l'activité de la protéine membranaire lytique ou on réprime l'expression de la protéine membranaire ou du gène de toxine à activité lytique, et, à un moment désiré, on supprime l'inhibition ou la répression.

4. Procédé selon les revendications 1 à 3, caractérisé en ce que l'on fait incuber la protéine avec un partenaire de liaison pour la protéine, éventuellement mis sous forme d'un dérivé, et on isole le conjugué formé lié à la membrane de bactéries gram-négatives.

5. Procédé selon la revendication 1, caractérisé en ce que la protéine de fusion contient au moins un autre domaine protéinique qui est constitué de 10 à 16 aminoacides et qui possède une structure secondaire repliée en ß.

6. Procédé selon la revendication 1, caractérisé en ce que l'on utilise pour le domaine protéinique les aminoacides 1 à 54 de la protéine E du phage PhiX174 ou les aminoacides 21 à 75 de la protéine L du phage MS2 et/ou une séquence d'aminoacides qui peut être obtenue à partir de ces séquences par échange d'aminoacides et qui possède une structure secondaire protéinique analogue.

7. Procédé selon la revendication 1, caractérisé en ce que les domaines protéiniques et la protéine recombinée sont liés par l'intermédiaire d'une séquence d'aminoacides hydrophile ayant 2 à 100 aminoacides et une structure secondaire désordonnée.

8. Procédé selon la revendication 1, caractérisé en ce que la protéine recombinée présente une structure antigénique.

9. Procédé selon la revendication 1, caractérisé en ce que la protéine recombinée est liée à un partenaire de liaison ne formant pas de liaison covalente avec cette protéine, auquel sont éventuellement liées d'autres substances de façon covalente ou non covalente.

10. Procédé selon la revendication 9, caractérisé en ce que, comme protéine recombinée, on utilise la streptavidine ou l'avidine.

11. Procédé selon les revendications 9 et 10, caractérisé en ce que le partenaire de liaison non covalente est un antigène biotinylé.

12. Procédé de préparation d'anticorps, caractérisé en ce que l'on immunise un mammifère avec une protéine qui a été obtenue selon les revendications 1 à 11, et on récupère les anticorps à partir du sérum ou de la rate.

13. Procédé de préparation d'anticorps selon la revendication 12, caractérisé en ce que l'on fusionne des lymphocytes B des animaux immunisés avec une lignée cellulaire convenable en présence d'agents de transformation, on clone et on cultive la lignée cellulaire productrice d'anticorps désirée et on récupère les anticorps à partir des cellules ou du surnageant de culture.

14. Utilisation des protéines liées à un support qui sont obtenues selon les revendications 1 à 11 pour la préparation de vaccins.
